# EUROPEAN PATENT APPLICATION

(11) **EP 3 054 009 A1**
(43) Date of publication of application: **10.08.2016**
(21) Application number: 14850475.6
(22) Date of filing: 01.10.2014
(51) Int. Cl.: C12N 15/09, C07K 1/22, C07K 19/00, C12N 1/15, C12N 1/19, C12N 1/21, C12N 5/10

(54) **PROTEIN COMPRISED BY LINKING BY LINKER MULTIPLE DOMAINS HAVING AFFINITY FOR PROTEINS HAVING Fc PART OF IMMUNOGLOBULIN G (IgG)**

(30) Priority: 04.10.2013 JP 2013209427
(71) Applicant: National Institute of Advanced Industrial Science and Technology, Tokyo 100-8921 (JP); Daicel Corporation, Osaka-shi, Osaka 530-0011 (JP)
(72) Inventor: HONDA Shinya, Tsukuba-shi Ibaraki 305-8566 (JP); WATANABE Hideki, Tsukuba-shi Ibaraki 305-8566 (JP); YOSHIDA Chuya, Tsukuba-shi Ibaraki 305-8566 (JP); ISOBE Yutaka, Himeji-shi Hyogo 671-1281 (JP); UEDA Momoko, Himeji-shi Hyogo 671-1281 (JP); NAKAI Yasuto, Himeji-shi Hyogo 671-1281 (JP)
(74) Representative: Power, David
(86) International application number: PCT/JP2014/076268
(87) International publication number: WO 2015/050153

(57) **Abstract**

The purpose of the present invention is to provide a novel protein, which have such excellent pH responsiveness that the antibody binding property to the Fc region of the immunoglobulin in the neutral region is improved and the binding property to the Fc region of the immunoglobulin in the weakly acidic region is much decreased when compared to the wild-type proteins such as protein G and protein A that have affinity for the protein comprising the Fc region of the immunoglobulin G (IgG), and a mutant-type protein (an improved protein) comprising their domain mutant.

The present invention relates to a protein constituted by linking plural domains having affinity for a protein comprising the Fc region of the immunoglobulin G (IgG) with linkers, wherein an extended length to the maximum of the linker is 80 -240 angstrom.

SEQUENCE LISTING

<110>  National Institute of Advanced Industrial science and Technology;
Daicel Corporation

<120>  Protein consisting of a plurality of domains having affinity for
protein having Fc portion of immunoglobulin G wherein the domains are bonded
via a linker

<130>  PCT-AB14027

<150>  JP 2013-209427
<151>  2013-10-04

<160>  46

<170>  PatentIn version 3.1

<210>  1
<211>  56
<212>  PRT
<213>  Streptcoccus sp.(Protein G B1domain; wild)

<400>  1

Asp Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  2
<211>  56
<212>  PRT
<213>  Streptcoccus sp.(Protein G B2domain; wild)

<400>  2

Thr Thr Tyr Lys Leu Val Ile Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  3
<211>  56
<212>  PRT
<213>  Streptcoccus sp.(Protein G B3domain; wild)

<400>  3

Thr Thr Tyr Lys Leu Val Ile Asn Gly Lys Thr Leu Lys Gly Glu Thr
1               5                   10                  15

Thr Thr Lys Ala Val Asp Ala Glu Thr Ala Glu Lys Ala Phe Lys Gln
            20                  25                  30

Tyr Ala Asn Asp Asn Gly Val Asp Gly Val Trp Thr Tyr Asp Asp Ala
        35                  40                  45

Thr Lys Thr Phe Thr Val Thr Glu
    50                  55

<210>  4
<211>  56
<212>  PRT
<213>  Artificial

<220>
<223>  ProteinG B1domain mutant

<220>
<221>  MISC_FEATURE
<222>  (35)..(35)
<223>  Xaa is Asn or Lys

<220>
<221>  MISC_FEATURE
<222>  (36)..(36)
<223>  Xaa is Asp or Glu

<220>
<221>  MISC_FEATURE
<222>  (37)..(37)
<223>  Xaa is Asn His or Leu

<220>
<221>  MISC_FEATURE
<222>  (47)..(47)
<223>  Xaa is Asp or Pro

<220>
<221>  MISC_FEATURE
<222>  (48)..(48)
<223>  Xaa is Ala, Lys or Glu

<220>

<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<220>
<221>  MISC_FEATURE
<222>  (42)..(42)
<223>  Xaa is Glu or His


<220>
<221>  MISC_FEATURE
<222>  (11)..(11)
<223>  Xaa is Thr or Arg


<220>
<221>  MISC_FEATURE
<222>  (17)..(17)
<223>  Xaa is Thr or Ile


<400>  4

Asp Thr Tyr Lys Leu Ile Leu Asn Gly Lys Xaa Leu Lys Gly Glu Thr
1               5                   10                  15


Xaa Thr Glu Ala Val Xaa Ala Ala Xaa Ala Glu Lys Val Phe Lys Xaa
            20                  25                  30


Tyr Ala Xaa Xaa Xaa Gly Val Xaa Gly Xaa Trp Thr Tyr Asp Xaa Xaa
        35                  40                  45


Thr Lys Thr Phe Thr Val Thr Glu
    50                  55


<210>  5
<211>  56
<212>  PRT
<213>  Artificial

<220>
<223>  ProteinG B2domain mutant

<220>
<221>  MISC_FEATURE
<222>  (35)..(35)
<223>  Xaa is Asn or Lys


<220>
<221>  MISC_FEATURE
<222>  (36)..(36)
<223>  Xaa is Asp or Glu


<220>
<221>  MISC_FEATURE
<222>  (37)..(37)
<223>  Xaa is Asn,His or Leu


<220>
<221>  MISC_FEATURE
<222>  (47)..(47)
<223>  Xaa is Asp or Pro


<220>
<221>  MISC_FEATURE
<222>  (48)..(48)
<223>  Xaa is Ala,Lys or Glu


<220>
<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<220>
<221>  MISC_FEATURE
<222>  (42)..(42)
<223>  Xaa is Glu or His

<220>
<221>  MISC_FEATURE
<222>  (11)..(11)
<223>  Xaa is Thr or Arg


<220>
<221>  MISC_FEATURE
<222>  (17)..(17)
<223>  Xaa is Thr or Ile


<400>  5

Thr Thr Tyr Lys Leu Val Ile Asn Gly Lys Xaa Leu Lys Gly Glu Thr
1               5                   10                  15


Xaa Thr Glu Ala Val Xaa Ala Ala Xaa Ala Glu Lys Val Phe Lys Xaa
            20                  25                  30


Tyr Ala Xaa Xaa Xaa Gly Val Xaa Gly Xaa Trp Thr Tyr Asp Xaa Xaa
        35                  40                  45


Thr Lys Thr Phe Thr Val Thr Glu
    50                  55


<210>  6
<211>  56
<212>  PRT
<213>  Artificial

<220>
<223>  ProteinG B3domain mutant

<220>
<221>  MISC_FEATURE
<222>  (35)..(35)
<223>  Xaa is Asn or Lys


<220>
<221>  MISC_FEATURE
<222>  (36)..(36)
<223>  Xaa is Asp or Glu


<220>
<221>  MISC_FEATURE
<222>  (37)..(37)
<223>  Xaa is Asn,His or Leu


<220>
<221>  MISC_FEATURE
<222>  (47)..(47)
<223>  Xaa is Asp or Pro

<220>
<221>  MISC_FEATURE
<222>  (48)..(48)
<223>  Xaa is Ala,Lys or Glu


<220>
<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<220>
<221>  MISC_FEATURE
<222>  (11)..(11)
<223>  Xaa is Thr or Arg


<220>
<221>  MISC_FEATURE
<222>  (17)..(17)
<223>  Xaa is Thr or Ile


<400>  6

Thr Thr Tyr Lys Leu Val Ile Asn Gly Lys Xaa Leu Lys Gly Glu Thr
1               5                   10                  15


Xaa Thr Lys Ala Val Xaa Ala Glu Xaa Ala Glu Lys Ala Phe Lys Xaa
            20                  25                  30


Tyr Ala Xaa Xaa Xaa Gly Val Xaa Gly Val Trp Thr Tyr Asp Xaa Xaa
        35                  40                  45


Thr Lys Thr Phe Thr Val Thr Glu
    50                  55


<210>  7
<211>  56
<212>  PRT

<213>  Artificial

<220>
<223>  ProteinG B1domain mutant

<220>
<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<220>
<221>  MISC_FEATURE
<222>  (42)..(42)
<223>  Xaa is Glu or His


<220>
<221>  MISC_FEATURE
<222>  (11)..(11)
<223>  Xaa is Thr or Arg


<220>
<221>  MISC_FEATURE
<222>  (17)..(17)
<223>  Xaa is Thr or Ile


<400>  7

Asp Thr Tyr Lys Leu Ile Leu Asn Gly Lys Xaa Leu Lys Gly Glu Thr
1               5                   10                  15


Xaa Thr Glu Ala Val Xaa Ala Ala Xaa Ala Glu Lys Val Phe Lys Xaa
            20                  25                  30


Tyr Ala Asn Asp Asn Gly Val Xaa Gly Xaa Trp Thr Tyr Asp Asp Ala
        35                  40                  45


Thr Lys Thr Phe Thr Val Thr Glu
    50                  55

<210>  8
<211>  56
<212>  PRT
<213>  Artificial

<220>
<223>  proteinG B2domain mutant

<220>
<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<220>
<221>  MISC_FEATURE
<222>  (42)..(42)
<223>  Xaa is Glu or His


<220>
<221>  MISC_FEATURE
<222>  (11)..(11)
<223>  Xaa is Thr or Arg


<220>
<221>  MISC_FEATURE
<222>  (17)..(17)
<223>  Xaa is Thr or Ile


<400>  8

Thr Thr Tyr Lys Leu Val Ile Asn Gly Lys Xaa Leu Lys Gly Glu Thr
1               5                   10                  15


Xaa Thr Glu Ala Val Xaa Ala Ala Xaa Ala Glu Lys Val Phe Lys Xaa
            20                  25                  30


Tyr Ala Asn Asp Asn Gly Val Xaa Gly Xaa Trp Thr Tyr Asp Asp Ala

35                  40                  45

Thr Lys Thr Phe Thr Val Thr Glu
    50                  55

<210>  9
<211>  56
<212>  PRT
<213>  Artificial

<220>
<223>  ProteinG B3domain mutant

<220>
<221>  MISC_FEATURE
<222>  (22)..(22)
<223>  Xaa is Asp or His

<220>
<221>  MISC_FEATURE
<222>  (25)..(25)
<223>  Xaa is Thr or His

<220>
<221>  MISC_FEATURE
<222>  (32)..(32)
<223>  Xaa is Gln or His

<220>
<221>  MISC_FEATURE
<222>  (40)..(40)
<223>  Xaa is Asp or His

<220>
<221>  MISC_FEATURE
<222>  (11)..(11)
<223>  Xaa is Thr or Arg

<220>
<221>  MISC_FEATURE
<222>  (17)..(17)
<223>  Xaa is Thr or Ile

<400>  9

Thr Thr Tyr Lys Leu Val Ile Asn Gly Lys Xaa Leu Lys Gly Glu Thr
1               5                   10                  15

Xaa Thr Lys Ala Val Xaa Ala Glu Xaa Ala Glu Lys Ala Phe Lys Xaa
            20                  25                  30

Tyr Ala Asn Asp Asn Gly Val Xaa Gly Val Trp Thr Tyr Asp Asp Ala
        35                  40                  45

Thr Lys Thr Phe Thr Val Thr Glu
    50                  55

<210>  10
<211>  56
<212>  PRT
<213>  Artificial

<220>
<223>  ProteinG B1domain mutant

<220>
<221>  MISC_FEATURE
<222>  (22)..(22)
<223>  Xaa is Asp or His

<220>
<221>  MISC_FEATURE
<222>  (25)..(25)
<223>  Xaa is Thr or His

<220>
<221>  MISC_FEATURE
<222>  (32)..(32)
<223>  Xaa is Gln or His

<220>
<221>  MISC_FEATURE
<222>  (40)..(40)
<223>  Xaa is Asp or His

<220>
<221>  MISC_FEATURE
<222>  (42)..(42)
<223>  Xaa is Glu or His

<400>  10

Asp Thr Tyr Lys Leu Ile Leu Asn Gly Lys Thr Leu Lys Gly Glu Thr
1               5                   10                  15

Thr Thr Glu Ala Val Xaa Ala Ala Xaa Ala Glu Lys Val Phe Lys Xaa
            20                  25                  30

Tyr Ala Asn Asp Asn Gly Val Xaa Gly Xaa Trp Thr Tyr Asp Asp Ala
        35                  40                  45

Thr Lys Thr Phe Thr Val Thr Glu
    50                  55

<210>  11

<211>  56
<212>  PRT
<213>  Artificial

<220>
<223>  ProteinG B2domain mutant

<220>
<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<220>
<221>  MISC_FEATURE
<222>  (42)..(42)
<223>  Xaa is Glu or His


<400>  11

Thr Thr Tyr Lys Leu Val Ile Asn Gly Lys Thr Leu Lys Gly Glu Thr
1               5                   10                  15


Thr Thr Glu Ala Val Xaa Ala Ala Xaa Ala Glu Lys Val Phe Lys Xaa
            20                  25                  30


Tyr Ala Asn Asp Asn Gly Val Xaa Gly Xaa Trp Thr Tyr Asp Asp Ala
        35                  40                  45


Thr Lys Thr Phe Thr Val Thr Glu
    50                  55


<210>  12
<211>  56
<212>  PRT
<213>  Artificial

<220>
<223>  ProteinG B3domain mutant

<220>
<221>  MISC_FEATURE
<222>  (22)..(22)
<223>  Xaa is Asp or His


<220>
<221>  MISC_FEATURE
<222>  (25)..(25)
<223>  Xaa is Thr or His


<220>
<221>  MISC_FEATURE
<222>  (32)..(32)
<223>  Xaa is Gln or His


<220>
<221>  MISC_FEATURE
<222>  (40)..(40)
<223>  Xaa is Asp or His


<400>  12

Thr Thr Tyr Lys Leu Val Ile Asn Gly Lys Thr Leu Lys Gly Glu Thr
1               5                   10                  15


Thr Thr Lys Ala Val Xaa Ala Glu Xaa Ala Glu Lys Ala Phe Lys Xaa
            20                  25                  30


Tyr Ala Asn Asp Asn Gly Val Xaa Gly Val Trp Thr Tyr Asp Asp Ala
        35                  40                  45


Thr Lys Thr Phe Thr Val Thr Glu
    50                  55


<210>  13
<211>  56
<212>  PRT
<213>  Artificial

<220>
<223>  ProteinG B1domain mutant

<400>  13

Asp Thr Tyr Lys Leu Ile Leu Asn Gly Lys Thr Leu Lys Gly Glu Thr
1               5                   10                  15


Thr Thr Glu Ala Val Asp Ala Ala Thr Ala Glu Lys Val Phe Lys Gln
            20                  25                  30


Tyr Ala Asn Glu His Gly Val Asp Gly Glu Trp Thr Tyr Asp Pro Glu
        35                  40                  45

Thr Lys Thr Phe Thr Val Thr Glu
    50                  55

<210>  14
<211>  56
<212>  PRT
<213>  Artificial

<220>
<223>  ProteinG B1domain mutant

<400>  14

Asp Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Glu His Gly Val His Gly Glu Trp Thr Tyr Asp Pro Glu
        35                  40                  45

Thr Lys Thr Phe Thr Val Thr Glu
    50                  55

<210>  15
<211>  56
<212>  PRT
<213>  Artificial

<220>
<223>  ProteinG B1domain mutant

<400>  15

Asp Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Glu His Gly Val Asp Gly His Trp Thr Tyr Asp Pro Glu
        35                  40                  45

Thr Lys Thr Phe Thr Val Thr Glu
    50                  55

<210>  16
<211>  56
<212>  PRT
<213>  Artificial

<220>

<223>  ProteinG B1domain mutant

<400>  16

Asp Thr Tyr Lys Leu Ile Leu Asn Gly Lys Arg Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Glu His Gly Val Asp Gly Glu Trp Thr Tyr Asp Pro Glu
        35                  40                  45

Thr Lys Thr Phe Thr Val Thr Glu
    50                  55

<210>  17
<211>  56
<212>  PRT
<213>  Artificial

<220>
<223>  ProteinG B1domain mutant

<400>  17

Asp Thr Tyr Lys Leu Ile Leu Asn Gly Lys Thr Leu Lys Gly Glu Thr
1               5                   10                  15

Ile Thr Glu Ala Val Asp Ala Ala Thr Ala Glu Lys Val Phe Lys Gln
            20                  25                  30

Tyr Ala Asn Glu His Gly Val Asp Gly Glu Trp Thr Tyr Asp Pro Glu
        35                  40                  45

Thr Lys Thr Phe Thr Val Thr Glu
    50                  55

<210>  18
<211>  56
<212>  PRT
<213>  Artificial

<220>
<223>  ProteinG B1domain mutant

<400>  18

Asp Thr Tyr Lys Leu Ile Leu Asn Gly Lys Arg Leu Lys Gly Glu Thr
1               5                   10                  15

Ile Thr Glu Ala Val Asp Ala Ala Thr Ala Glu Lys Val Phe Lys Gln
            20                  25                  30

Tyr Ala Asn Glu His Gly Val Asp Gly Glu Trp Thr Tyr Asp Pro Glu
        35                  40                  45

Thr Lys Thr Phe Thr Val Thr Glu
    50                  55

<210>  19
<211>  56
<212>  PRT
<213>  Artificial

<220>
<223>  ProteinG B1domain mutant

<400>  19

Asp Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys His
            20                  25                  30

Tyr Ala Asn Glu His Gly Val His Gly His Trp Thr Tyr Asp Pro Glu
        35                  40                  45

Thr Lys Thr Phe Thr Val Thr Glu
    50                  55

<210>  20
<211>  56
<212>  PRT
<213>  Artificial

<220>
<223>  ProteinG B1domain mutant

<400>  20

Asp Thr Tyr Lys Leu Ile Leu Asn Gly Lys Thr Leu Lys Gly Glu Thr
1               5                   10                  15

Thr Thr Glu Ala Val His Ala Ala Thr Ala Glu Lys Val Phe Lys His
            20                  25                  30

Tyr Ala Asn Glu His Gly Val His Gly His Trp Thr Tyr Asp Pro Glu
        35                  40                  45

Thr Lys Thr Phe Thr Val Thr Glu
    50                  55

<210>  21
<211>  168

<212>  DNA
<213>  Polynucleotide encoding Streptcoccus sp. Protein G B1domain

<400>  21
gacacttaca aattaatcct taatggtaaa acattgaaag gcgaaacaac tactgaagct      60

gttgatgctg ctactgcaga aaaagtcttc aaacaatacg ctaacgacaa cggtgttgac     120

ggtgaatgga cttacgacga tgcgactaag acctttacag ttactgaa                  168


<210>  22
<211>  168
<212>  DNA
<213>  Artificial

<220>
<223>  Polynucleotide encoding protein G B1domain mutant

<400>  22
gacacttaca aattaatcct taatggtaaa acattgaaag gcgaaacaac tactgaagct      60

gttgatgctg ctactgcaga aaaagtcttc aaacaatacg ctaacgaaca tggtgttgac     120

ggtgaatgga cttacgaccc ggaaactaag acctttacag ttactgaa                  168


<210>  23
<211>  168
<212>  DNA
<213>  Artificial

<220>
<223>  Polynucleotide encoding protein G B1domain mutant

<400>  23
gatacctaca aactgatcct gaatggtaaa accctgaaag gcgaaaccac cactgaggcg      60

gtagacgctg cgaccgcgga gaaagttttc aaacagtacg ctaacgaaca cggtgttcac     120

ggcgagtgga cctacgaccc ggaaaccaag accttcaccg ttaccgaa                  168


<210>  24
<211>  168
<212>  DNA
<213>  Artificial

<220>
<223>  Polynucleotide encoding protein G B1domain mutant

<400>  24
gatacctaca aactgatcct gaatggtaaa accctgaaag gcgaaaccac cactgaggcg      60

gtagacgctg cgaccgcgga gaaagttttc aaacagtacg ctaacgaaca cggtgttgac     120

ggccactgga cctacgaccc ggaaaccaag accttcaccg ttaccgaa                  168


<210>  25
<211>  168
<212>  DNA
<213>  Artificial

<220>
<223>  Polynucleotide encoding protein G B1domain mutant

<400>  25
gatacctaca aactgatcct gaatggtaaa cgtctgaaag gcgaaaccac cactgaggcg      60

gtagacgctg cgaccgcgga gaaagttttc aaacagtacg ctaacgaaca cggtgttgac     120

ggcgagtgga cctacgaccc ggaaaccaag accttcaccg ttaccgaa                  168


<210>  26
<211>  168
<212>  DNA
<213>  Artificial

<220>
<223>  Polynucleotide encoding protein G B1domain mutant

<400>  26
gatacctaca aactgatcct gaatggtaaa accctgaaag gcgaaaccat caccgaggcg      60

gtagacgctg cgaccgcgga gaaagttttc aaacagtacg ctaacgaaca cggtgttgac     120

ggcgagtgga cctacgaccc ggaaaccaag accttcaccg ttaccgaa                  168


<210>  27
<211>  168
<212>  DNA
<213>  Artificial

<220>
<223>  Polynucleotide encoding protein G B1domain mutant

<400>  27
gatacctaca aactgatcct gaatggtaaa cgcctgaaag gcgaaaccat caccgaggcg      60

gtagacgctg cgaccgcgga gaaagttttc aaacagtacg ctaacgaaca cggtgttgac     120

ggcgagtgga cctacgaccc ggaaaccaag accttcaccg ttaccgaa                  168


<210>  28
<211>  168
<212>  DNA
<213>  Artificial

<220>
<223>  Polynucleotide encoding protein G B1domain mutant

<400>  28
gatacctaca aactgatcct gaatggtaaa cgcctgaaag gcgaaaccat caccgaggcg      60

gtagacgctg cgaccgcgga gaaagttttc aaacagtacg ctaacgaaca cggtgttgac     120

ggcgagtgga cctacgaccc ggaaaccaag accttcaccg ttaccgaa                  168


<210>  29
<211>  168
<212>  DNA

<213>  Artificial

<220>
<223>  Polynucleotide encoding protein G B1domain mutant

<400>  29
gatacctaca aactgatcct gaatggtaaa accctgaaag gcgaaaccac cactgaggcg      60

gtacacgctg cgaccgcgga gaaagttttc aaacactacg ctaacgaaca cggtgttcac     120

ggccactgga cctacgaccc ggaaaccaag accttcaccg ttaccgaa                  168


<210>  30
<211>  2456
<212>  DNA
<213>  Streptcoccus sp.G148(polynucleotide encoding Protein G; wild)

<400>  30
gaattctcta ttataaataa aataaatagt actatagata gaaaatctca tttttaaaaa      60

gtcttgtttt cttaaagaag aaaataattg ttgaaaaatt atagaaaatc atttttatac     120

taatgaaata aacataaggc taaattggtc aggtgatgat aggagattta tttgtaagga     180

ttccttaatt ttattaatta acaaaaattg atagaaaaat taaatgaaat ccttgattta     240

attttattaa gttgtataat aaaaagtgaa attattaaat cgtagtttca aatttgtcgg     300

ctttttaata tgtgctggca tattaaaaat aaaaaaggag aaaaaatgga aaaagaaaaa     360

aaggtaaaat actttttacg taaatcagct tttgggttag catccgtatc agctgcattt     420

ttagtgggat caacggtatt cgctgttgac tcaccaatcg aagatacccc aattattcgt     480

aatggtggtg aattaactaa tcttctgggg aattcagaga caacactggc tttgcgtaat     540

gaagagagtg ctacagctga tttgacagca gcagcggtag ccgatactgt ggcagcagcg     600

gcagctgaaa atgctggggc agcagcttgg gaagcagcgg cagcagcaga tgctctagca     660

aaagccaaag cagatgccct taaagaattc aacaaatatg gagtaagtga ctattacaag     720

aatctaatca acaatgccaa aactgttgaa ggcgtaaaag accttcaagc acaagttgtt     780

gaatcagcga agaaagcgcg tatttcagaa gcaacagatg gcttatctga tttcttgaaa     840

tcacaaacac ctgctgaaga tactgttaaa tcaattgaat tagctgaagc taaagtctta     900

gctaacagag aacttgacaa atatggagta agtgactatc acaagaacct aatcaacaat     960

gccaaaactg ttgaaggtgt aaaagacctt caagcacaag ttgttgaatc agcgaagaaa    1020

gcgcgtattt cagaagcaac agatggctta tctgatttct tgaaatcaca aacacctgct    1080

gaagatactg ttaaatcaat tgaattagct gaagctaaag tcttagctaa cagagaactt    1140

gacaaatatg gagtaagtga ctattacaag aacctaatca acaatgccaa aactgttgaa    1200

ggtgtaaaag cactgataga tgaaatttta gctgcattac ctaagactga cacttacaaa    1260

ttaatcctta atggtaaaac attgaaaggc gaaacaacta ctgaagctgt tgatgctgct    1320

actgcagaaa aagtcttcaa acaatacgct aacgacaacg gtgttgacgg tgaatggact 1380

tacgacgatg cgactaagac ctttacagtt actgaaaaac cagaagtgat cgatgcgtct 1440

gaattaacac cagccgtgac aacttacaaa cttgttatta atggtaaaac attgaaaggc 1500

gaaacaacta ctgaagctgt tgatgctgct actgcagaaa aagtcttcaa acaatacgct 1560

aacgacaacg gtgttgacgg tgaatggact tacgacgatg cgactaagac ctttacagtt 1620

actgaaaaac cagaagtgat cgatgcgtct gaattaacac cagccgtgac aacttacaaa 1680

cttgttatta atggtaaaac attgaaaggc gaaacaacta ctaaagcagt agacgcagaa 1740

actgcagaaa aagccttcaa acaatacgct aacgacaacg gtgttgatgg tgtttggact 1800

tatgatgatg cgactaagac ctttacggta actgaaatgg ttacagaggt tcctggtgat 1860

gcaccaactg aaccagaaaa accagaagca agtatccctc ttgttccgtt aactcctgca 1920

actccaattg ctaaagatga cgctaagaaa gacgatacta agaaagaaga tgctaaaaaa 1980

ccagaagcta agaaagaaga cgctaagaaa gctgaaactc ttcctacaac tggtgaagga 2040

agcaacccat tcttcacagc agctgcgctt gcagtaatgg ctggggcggg tgctttggcg 2100

gtcgcttcaa aacgtaaaga agactaattg tcattatttt tgacaaaaag ctttttaaga 2160

ggaacactag ggttcctctt tttttgtatt tttaaaaaca caagtaatac agttgacagc 2220

tatttctcta aggatggtgg aaaggatagg acatctaagt cctgaaaata gtagtttttg 2280

caaaaaaagc tcacagaaag ctaaaaactg ttatagaata tttctacgat atttgttata 2340

ataaaactaa tacaggaaaa gtggaaaggg catcaatgga tatttggaca cagttggcag 2400

catttgcttt tttggacact ccaaagttgc attgaggcct tttcaatatg ccgatc 2456

<210> 31
<211> 103
<212> PRT
<213> Klebsiella pneeumoniae (C-terminal sequense oxaloacetate
decarboxylase alpha-subunit c-terminal domain)

<400> 31

Met Gly Ala Gly Thr Pro Val Thr Ala Pro Leu Ala Gly Thr Ile Trp
1               5                   10                  15

Lys Val Leu Ala Ser Glu Gly Gln Thr Val Ala Ala Gly Glu Val Leu
            20                  25                  30

Leu Ile Leu Glu Ala Met Lys Met Glu Thr Glu Ile Arg Ala Ala Gln
        35                  40                  45

Ala Gly Thr Val Arg Gly Ile Ala Val Lys Ala Gly Asp Ala Val Ala
    50                  55                  60

Val Gly Asp Thr Leu Met Thr Leu Ala Gly Ser Gly Ser Asp Leu Tyr
65                  70                  75                  80

Asp Asp Asp Asp Lys Gly Ile Ile Thr Ser Leu Tyr Lys Lys Ala Gly
                85                  90                  95

Ser Ala Ala Ala Pro Phe Thr
            100

<210>  32
<211>  30
<212>  DNA
<213>  Artificial

<220>
<223>  Primer

<400>  32
atagctccat ggacacttac aaattaatcc                                        30

<210>  33
<211>  30
<212>  DNA
<213>  Artificial

<220>
<223>  Primer

<400>  33
attggatcct tattcagtaa ctgtaaaggt                                        30

<210>  34
<211>  30
<212>  DNA
<213>  Artificial

<220>
<223>  Primer

<400>  34
atagctccat ggatacctac aaactgatcc                                        30

<210>  35
<211>  30
<212>  DNA
<213>  Artificial

<220>
<223>  Primer

<400>  35
attggatcct tattcggtaa cggtgaaggt                                        30

<210>  36
<211>  711
<212>  DNA

<213>  Artificial Sequence

<220>
<223>  CGB01H-3D

<220>
<221>  CDS
<222>  (1)..(711)
<223>

<400>  36
atg gcg tat tac gat cca gaa acg ggc act tgg tat tct ggc ggt cgt      48
Met Ala Tyr Tyr Asp Pro Glu Thr Gly Thr Trp Tyr Ser Gly Gly Arg
1               5                   10                  15

gat acc tat aaa ctg att ctg aat ggt aaa acc ctg aaa ggt gaa acc      96
Asp Thr Tyr Lys Leu Ile Leu Asn Gly Lys Thr Leu Lys Gly Glu Thr
            20                  25                  30

acc acc gaa gca gtt gat gca gca acc gca gaa aaa gtt ttt aaa caa      144
Thr Thr Glu Ala Val Asp Ala Ala Thr Ala Glu Lys Val Phe Lys Gln
        35                  40                  45

tat gcc aat gac aac ggt gtt gac ggt gaa tgg acc tat gat gat gcg      192
Tyr Ala Asn Asp Asn Gly Val Asp Gly Glu Trp Thr Tyr Asp Asp Ala
    50                  55                  60

acc aaa acc ttt acc gtt acc gaa cgt ccg gaa gtt att gat gca agc      240
Thr Lys Thr Phe Thr Val Thr Glu Arg Pro Glu Val Ile Asp Ala Ser
65                  70                  75                  80

gaa ctg acc cca gca gtg gat aca tat aag ctg atc ctg aac ggc aag      288
Glu Leu Thr Pro Ala Val Asp Thr Tyr Lys Leu Ile Leu Asn Gly Lys
                85                  90                  95

aca ctg aag ggc gaa acc aca act gaa gcc gtg gat gcc gcc acc gcc      336
Thr Leu Lys Gly Glu Thr Thr Thr Glu Ala Val Asp Ala Ala Thr Ala
            100                 105                 110

gaa aag gtg ttt aag cag tac gca aac gat aat ggc gtg gat ggc gag      384
Glu Lys Val Phe Lys Gln Tyr Ala Asn Asp Asn Gly Val Asp Gly Glu
        115                 120                 125

tgg aca tat gat gac gcc aca aag aca ttt acc gtg aca gaa cgc cct      432
Trp Thr Tyr Asp Asp Ala Thr Lys Thr Phe Thr Val Thr Glu Arg Pro
    130                 135                 140

gaa gtg atc gat gca tct gaa ctg aca ccg gcc gtt gat act tat aaa      480
Glu Val Ile Asp Ala Ser Glu Leu Thr Pro Ala Val Asp Thr Tyr Lys
145                 150                 155                 160

ctg atc ctg aat gga aag act ctg aaa gga gag aca acc acg gaa gcg      528
Leu Ile Leu Asn Gly Lys Thr Leu Lys Gly Glu Thr Thr Thr Glu Ala
                165                 170                 175

gtg gat gcg gcg aca gcg gaa aaa gta ttc aag cag tat gcg aac gat      576
Val Asp Ala Ala Thr Ala Glu Lys Val Phe Lys Gln Tyr Ala Asn Asp
            180                 185                 190

aac ggc gta gat ggc gaa tgg act tat gat gac gcg act aaa acg ttc      624
Asn Gly Val Asp Gly Glu Trp Thr Tyr Asp Asp Ala Thr Lys Thr Phe
        195                 200                 205

aca gtg acc gag cgc cct gag gtt att gat gcc tca gag ctg acc ccg 672
Thr Val Thr Glu Arg Pro Glu Val Ile Asp Ala Ser Glu Leu Thr Pro
    210                 215                 220

gct gtt ggt ggt ggt ggt tgt cat cat cat cac cat cat 711
Ala Val Gly Gly Gly Gly Cys His His His His His His
225                 230                 235

<210> 37
<211> 711
<212> DNA
<213> Artificial Sequence

<220>
<223> CGB19H-3D

<220>
<221> CDS
<222> (1)..(711)
<223>

<400> 37
atg gcg tat tac gat cca gaa acg ggc act tgg tat tct ggc ggt cgt 48
Met Ala Tyr Tyr Asp Pro Glu Thr Gly Thr Trp Tyr Ser Gly Gly Arg
1               5                   10                  15

gat acc tat aaa ctg att ctg aat ggt aaa acc ctg aaa ggt gaa acc 96
Asp Thr Tyr Lys Leu Ile Leu Asn Gly Lys Thr Leu Lys Gly Glu Thr
            20                  25                  30

acc acc gaa gca gtt gat gca gca acc gca gaa aaa gtt ttt aaa cat 144
Thr Thr Glu Ala Val Asp Ala Ala Thr Ala Glu Lys Val Phe Lys His
        35                  40                  45

tat gcc aat gaa cat ggt gtt cat ggt cat tgg acc tat gat ccg gaa 192
Tyr Ala Asn Glu His Gly Val His Gly His Trp Thr Tyr Asp Pro Glu
    50                  55                  60

acc aaa acc ttt acc gtt acc gaa cgt ccg gaa gtt att gat gca agc 240
Thr Lys Thr Phe Thr Val Thr Glu Arg Pro Glu Val Ile Asp Ala Ser
65                  70                  75                  80

gaa ctg acc cca gca gtg gat aca tat aag ctg atc ctg aac ggc aag 288
Glu Leu Thr Pro Ala Val Asp Thr Tyr Lys Leu Ile Leu Asn Gly Lys
                85                  90                  95

aca ctg aag ggc gaa acc aca act gaa gcc gtg gat gcc gcc acc gcc 336
Thr Leu Lys Gly Glu Thr Thr Thr Glu Ala Val Asp Ala Ala Thr Ala
            100                 105                 110

gaa aag gtg ttt aag cac tac gca aac gaa cat ggc gtg cac ggc cac 384
Glu Lys Val Phe Lys His Tyr Ala Asn Glu His Gly Val His Gly His
        115                 120                 125

tgg aca tat gat cct gag aca aag aca ttt acc gtg aca gaa cgc cct 432
Trp Thr Tyr Asp Pro Glu Thr Lys Thr Phe Thr Val Thr Glu Arg Pro
    130                 135                 140

gaa gtg atc gat gca tct gaa ctg aca ccg gcc gtt gat act tat aaa 480
Glu Val Ile Asp Ala Ser Glu Leu Thr Pro Ala Val Asp Thr Tyr Lys
145                 150                 155                 160

ctg atc ctg aat gga aag act ctg aaa gga gag aca acc acg gaa gcg     528
Leu Ile Leu Asn Gly Lys Thr Leu Lys Gly Glu Thr Thr Thr Glu Ala
                165                 170                 175

gtg gat gcg gcg aca gcg gaa aaa gta ttc aag cat tat gcg aac gag     576
Val Asp Ala Ala Thr Ala Glu Lys Val Phe Lys His Tyr Ala Asn Glu
            180                 185                 190

cac ggc gta cat ggc cat tgg act tat gat cca gag act aaa acg ttc     624
His Gly Val His Gly His Trp Thr Tyr Asp Pro Glu Thr Lys Thr Phe
        195                 200                 205

aca gtg acc gag cgc cct gag gtt att gat gcc tca gag ctg acc ccg     672
Thr Val Thr Glu Arg Pro Glu Val Ile Asp Ala Ser Glu Leu Thr Pro
    210                 215                 220

gct gtt ggt ggt ggt ggt tgt cat cat cat cac cat cat                 711
Ala Val Gly Gly Gly Gly Cys His His His His His His
225                 230                 235

<210>  38
<211>  339
<212>  DNA
<213>  Artificial Sequence

<220>
<223>  CGB19H-1D

<220>
<221>  CDS
<222>  (1)..(336)
<223>

<400>  38
atg gcg tat tac gat cca gaa acg ggc act tgg tat tct ggc ggt cgt      48
Met Ala Tyr Tyr Asp Pro Glu Thr Gly Thr Trp Tyr Ser Gly Gly Arg
1               5                   10                  15

gat acc tat aaa ctg att ctg aat ggt aaa acc ctg aaa ggt gaa acc      96
Asp Thr Tyr Lys Leu Ile Leu Asn Gly Lys Thr Leu Lys Gly Glu Thr
            20                  25                  30

acc acc gaa gca gtt gat gca gca acc gca gaa aaa gtt ttt aaa cat     144
Thr Thr Glu Ala Val Asp Ala Ala Thr Ala Glu Lys Val Phe Lys His
        35                  40                  45

tat gcc aat gaa cat ggt gtt cat ggt cat tgg acc tat gat ccg gaa     192
Tyr Ala Asn Glu His Gly Val His Gly His Trp Thr Tyr Asp Pro Glu
    50                  55                  60

acc aaa acc ttt acc gtt acc gaa cgt ccg gaa gtt att gat gca agc     240
Thr Lys Thr Phe Thr Val Thr Glu Arg Pro Glu Val Ile Asp Ala Ser
65                  70                  75                  80

gaa ctg acc cca gca gtg ggt ggt ggt ggt agt ggt ggt ggt ggt agt     288
Glu Leu Thr Pro Ala Val Gly Gly Gly Gly Ser Gly Gly Gly Gly Ser
                85                  90                  95

ggt ggt ggt ggt agt ggt ggt ggt ggt tgt cat cat cat cac cat cat     336
Gly Gly Gly Gly Ser Gly Gly Gly Gly Cys His His His His His His
            100                 105                 110

taa                                                                   339

<210>  39
<211>  969
<212>  DNA
<213>  Artificial Sequence

<220>
<223>  CGB19H-4D

<220>
<221>  CDS
<222>  (1)..(966)
<223>

<400>  39
atg gcg tat tac gat cca gaa acg ggc act tgg tat tct ggc ggt cgt       48
Met Ala Tyr Tyr Asp Pro Glu Thr Gly Thr Trp Tyr Ser Gly Gly Arg
1               5                   10                  15

gat acc tat aaa ctg att ctg aat ggt aaa acc ctg aaa ggt gaa acc       96
Asp Thr Tyr Lys Leu Ile Leu Asn Gly Lys Thr Leu Lys Gly Glu Thr
            20                  25                  30

acc acc gaa gca gtt gat gca gca acc gca gaa aaa gtt ttt aaa cat      144
Thr Thr Glu Ala Val Asp Ala Ala Thr Ala Glu Lys Val Phe Lys His
        35                  40                  45

tat gcc aat gaa cat ggt gtt cat ggt cat tgg acc tat gat ccg gaa      192
Tyr Ala Asn Glu His Gly Val His Gly His Trp Thr Tyr Asp Pro Glu
    50                  55                  60

acc aaa acc ttt acc gtt acc gaa cgt ccg gaa gtt att gat gca agc      240
Thr Lys Thr Phe Thr Val Thr Glu Arg Pro Glu Val Ile Asp Ala Ser
65                  70                  75                  80

gaa ctg acc cca gca gtg gat aca tat aag ctg atc ctg aac ggc aag      288
Glu Leu Thr Pro Ala Val Asp Thr Tyr Lys Leu Ile Leu Asn Gly Lys
                85                  90                  95

aca ctg aag ggc gaa acc aca act gaa gcc gtg gat gcc gcc acc gcc      336
Thr Leu Lys Gly Glu Thr Thr Thr Glu Ala Val Asp Ala Ala Thr Ala
            100                 105                 110

gaa aag gtg ttt aag cac tac gca aac gaa cat ggc gtg cac ggc cac      384
Glu Lys Val Phe Lys His Tyr Ala Asn Glu His Gly Val His Gly His
        115                 120                 125

tgg aca tat gat cct gag aca aag aca ttt acc gtg aca gaa cgc cct      432
Trp Thr Tyr Asp Pro Glu Thr Lys Thr Phe Thr Val Thr Glu Arg Pro
    130                 135                 140

gaa gtg atc gat gca tct gaa ctg aca ccg gcc gtt gat act tat aaa      480
Glu Val Ile Asp Ala Ser Glu Leu Thr Pro Ala Val Asp Thr Tyr Lys
145                 150                 155                 160

ctg atc ctg aat gga aag act ctg aaa gga gag aca acc acg gaa gcg      528
Leu Ile Leu Asn Gly Lys Thr Leu Lys Gly Glu Thr Thr Thr Glu Ala
                165                 170                 175

gtg gat gcg gcg aca gcg gaa aaa gta ttc aag cat tat gcg aac gag     576
Val Asp Ala Ala Thr Ala Glu Lys Val Phe Lys His Tyr Ala Asn Glu
            180                 185                 190

cac ggc gta cat ggc cat tgg act tat gat cca gag act aaa acg ttc     624
His Gly Val His Gly His Trp Thr Tyr Asp Pro Glu Thr Lys Thr Phe
        195                 200                 205

aca gtg acc gag cgc cct gag gtt att gat gcc tca gag ctg acc ccg     672
Thr Val Thr Glu Arg Pro Glu Val Ile Asp Ala Ser Glu Leu Thr Pro
    210                 215                 220

gct gtt gat acc tat aaa ctg att ctg aat ggt aaa acc ctg aaa ggt     720
Ala Val Asp Thr Tyr Lys Leu Ile Leu Asn Gly Lys Thr Leu Lys Gly
225                 230                 235                 240

gaa acc acc acc gaa gca gtt gat gca gca acc gca gaa aaa gtt ttt     768
Glu Thr Thr Thr Glu Ala Val Asp Ala Ala Thr Ala Glu Lys Val Phe
                245                 250                 255

aaa cat tat gcc aat gaa cat ggt gtt cat ggt cat tgg acc tat gat     816
Lys His Tyr Ala Asn Glu His Gly Val His Gly His Trp Thr Tyr Asp
            260                 265                 270

ccg gaa acc aaa acc ttt acc gtt acc gaa cgt ccg gaa gtt att gat     864
Pro Glu Thr Lys Thr Phe Thr Val Thr Glu Arg Pro Glu Val Ile Asp
        275                 280                 285

gca agc gaa ctg acc cca gca gtg ggt ggt ggt ggt agt ggt ggt ggt     912
Ala Ser Glu Leu Thr Pro Ala Val Gly Gly Gly Gly Ser Gly Gly Gly
    290                 295                 300

ggt agt ggt ggt ggt ggt agt ggt ggt ggt ggt tgt cat cat cat cac     960
Gly Ser Gly Gly Gly Gly Ser Gly Gly Gly Gly Cys His His His His
305                 310                 315                 320

cat cat taa                                                         969
His His


<210>  40
<211>  1179
<212>  DNA
<213>  Artificial Seqeunce

<220>
<223>  CGB19H-5D

<220>
<221>  CDS
<222>  (1)..(1176)
<223>

<400>  40
atg gcg tat tac gat cca gaa acg ggc act tgg tat tct ggc ggt cgt      48
Met Ala Tyr Tyr Asp Pro Glu Thr Gly Thr Trp Tyr Ser Gly Gly Arg
1               5                   10                  15

gat acc tat aaa ctg att ctg aat ggt aaa acc ctg aaa ggt gaa acc      96
Asp Thr Tyr Lys Leu Ile Leu Asn Gly Lys Thr Leu Lys Gly Glu Thr
            20                  25                  30

acc acc gaa gca gtt gat gca gca acc gca gaa aaa gtt ttt aaa cat     144

Thr Thr Glu Ala Val Asp Ala Ala Thr Ala Glu Lys Val Phe Lys His
        35                  40                  45

tat gcc aat gaa cat ggt gtt cat ggt cat tgg acc tat gat ccg gaa      192
Tyr Ala Asn Glu His Gly Val His Gly His Trp Thr Tyr Asp Pro Glu
    50                  55                  60

acc aaa acc ttt acc gtt acc gaa cgt ccg gaa gtt att gat gca agc      240
Thr Lys Thr Phe Thr Val Thr Glu Arg Pro Glu Val Ile Asp Ala Ser
65                  70                  75                  80

gaa ctg acc cca gca gtg gat aca tat aag ctg atc ctg aac ggc aag      288
Glu Leu Thr Pro Ala Val Asp Thr Tyr Lys Leu Ile Leu Asn Gly Lys
                85                  90                  95

aca ctg aag ggc gaa acc aca act gaa gcc gtg gat gcc gcc acc gcc      336
Thr Leu Lys Gly Glu Thr Thr Thr Glu Ala Val Asp Ala Ala Thr Ala
            100                 105                 110

gaa aag gtg ttt aag cac tac gca aac gaa cat ggc gtg cac ggc cac      384
Glu Lys Val Phe Lys His Tyr Ala Asn Glu His Gly Val His Gly His
        115                 120                 125

tgg aca tat gat cct gag aca aag aca ttt acc gtg aca gaa cgc cct      432
Trp Thr Tyr Asp Pro Glu Thr Lys Thr Phe Thr Val Thr Glu Arg Pro
    130                 135                 140

gaa gtg atc gat gca tct gaa ctg aca ccg gcc gtt gat act tat aaa      480
Glu Val Ile Asp Ala Ser Glu Leu Thr Pro Ala Val Asp Thr Tyr Lys
145                 150                 155                 160

ctg atc ctg aat gga aag act ctg aaa gga gag aca acc acg gaa gcg      528
Leu Ile Leu Asn Gly Lys Thr Leu Lys Gly Glu Thr Thr Thr Glu Ala
                165                 170                 175

gtg gat gcg gcg aca gcg gaa aaa gta ttc aag cat tat gcg aac gag      576
Val Asp Ala Ala Thr Ala Glu Lys Val Phe Lys His Tyr Ala Asn Glu
            180                 185                 190

cac ggc gta cat ggc cat tgg act tat gat cca gag act aaa acg ttc      624
His Gly Val His Gly His Trp Thr Tyr Asp Pro Glu Thr Lys Thr Phe
        195                 200                 205

aca gtg acc gag cgc cct gag gtt att gat gcc tca gag ctg acc ccg      672
Thr Val Thr Glu Arg Pro Glu Val Ile Asp Ala Ser Glu Leu Thr Pro
    210                 215                 220

gct gtt gat acc tat aaa ctg att ctg aat ggt aaa acc ctg aaa ggt      720
Ala Val Asp Thr Tyr Lys Leu Ile Leu Asn Gly Lys Thr Leu Lys Gly
225                 230                 235                 240

gaa acc acc acc gaa gca gtt gat gca gca acc gca gaa aaa gtt ttt      768
Glu Thr Thr Thr Glu Ala Val Asp Ala Ala Thr Ala Glu Lys Val Phe
                245                 250                 255

aaa cat tat gcc aat gaa cat ggt gtt cat ggt cat tgg acc tat gat      816
Lys His Tyr Ala Asn Glu His Gly Val His Gly His Trp Thr Tyr Asp
            260                 265                 270

ccg gaa acc aaa acc ttt acc gtt acc gaa cgt ccg gaa gtt att gat      864
Pro Glu Thr Lys Thr Phe Thr Val Thr Glu Arg Pro Glu Val Ile Asp
        275                 280                 285

gca agc gaa ctg acc cca gca gtg gat aca tat aag ctg atc ctg aac 912
Ala Ser Glu Leu Thr Pro Ala Val Asp Thr Tyr Lys Leu Ile Leu Asn
    290                 295                 300

ggc aag aca ctg aag ggc gaa acc aca act gaa gcc gtg gat gcc gcc 960
Gly Lys Thr Leu Lys Gly Glu Thr Thr Thr Glu Ala Val Asp Ala Ala
305                 310                 315                 320

acc gcc gaa aag gtg ttt aag cac tac gca aac gaa cat ggc gtg cac 1008
Thr Ala Glu Lys Val Phe Lys His Tyr Ala Asn Glu His Gly Val His
                325                 330                 335

ggc cac tgg aca tat gat cct gag aca aag aca ttt acc gtg aca gaa 1056
Gly His Trp Thr Tyr Asp Pro Glu Thr Lys Thr Phe Thr Val Thr Glu
            340                 345                 350

cgc cct gaa gtg atc gat gca tct gaa ctg aca ccg gcc gtt ggt ggt 1104
Arg Pro Glu Val Ile Asp Ala Ser Glu Leu Thr Pro Ala Val Gly Gly
        355                 360                 365

ggt ggt agt ggt ggt ggt ggt agt ggt ggt ggt ggt agt ggt ggt ggt 1152
Gly Gly Ser Gly Gly Gly Gly Ser Gly Gly Gly Gly Ser Gly Gly Gly
    370                 375                 380

ggt tgt cat cat cat cac cat cat taa 1179
Gly Cys His His His His His His
385                 390

<210> 41
<211> 228
<212> PRT
<213> Artificial Sequence

<220>
<223> PG2LL10

<400> 41

Met Ala Tyr Tyr Asp Pro Glu Thr Gly Thr Trp Tyr Ser Gly Gly Arg
1               5                   10                  15

Asp Thr Tyr Lys Leu Ile Leu Asn Gly Lys Thr Leu Lys Gly Glu Thr
            20                  25                  30

Thr Thr Glu Ala Val Asp Ala Ala Thr Ala Glu Lys Val Phe Lys His
        35                  40                  45

Tyr Ala Asn Glu His Gly Val His Gly His Trp Thr Tyr Asp Pro Glu
    50                  55                  60

Thr Lys Thr Phe Thr Val Thr Glu Gly Gly Ser Gly Gly Ser Gly Gly
65                  70                  75                  80

Ser Gly Gly Ser Gly Gly Ser Gly Gly Ser Gly Gly Ser Gly Gly Ser
                85                  90                  95

Gly Gly Ser Gly Gly Ser Gly Gly Ser Gly Gly Ser Gly Gly Ser Gly
            100                 105                 110

Gly Ser Gly Gly Ser Gly Gly Ser Gly Gly Ser Gly Gly Ser Gly Gly
        115                 120                 125

Ser Gly Gly Ser Asp Thr Tyr Lys Leu Ile Leu Asn Gly Lys Thr Leu
    130                 135                 140

Lys Gly Glu Thr Thr Thr Glu Ala Val Asp Ala Ala Thr Ala Glu Lys
145                 150                 155                 160

Val Phe Lys His Tyr Ala Asn Glu His Gly Val His Gly His Trp Thr
                165                 170                 175

Tyr Asp Pro Glu Thr Lys Thr Phe Thr Val Thr Glu Arg Pro Glu Val
            180                 185                 190

Ile Asp Ala Ser Glu Leu Thr Pro Ala Val Gly Gly Gly Gly Ser Gly
        195                 200                 205

Gly Gly Gly Ser Gly Gly Gly Gly Ser Gly Gly Gly Gly Cys His His
    210                 215                 220

His His His His
225

<210>  42
<211>  210
<212>  PRT
<213>  Artificial Sequence

<220>
<223>  PG2LL7

<400>  42

Met Ala Tyr Tyr Asp Pro Glu Thr Gly Thr Trp Tyr Ser Gly Gly Arg
1               5                   10                  15

Asp Thr Tyr Lys Leu Ile Leu Asn Gly Lys Thr Leu Lys Gly Glu Thr
            20                  25                  30

Thr Thr Glu Ala Val Asp Ala Ala Thr Ala Glu Lys Val Phe Lys His
        35                  40                  45

Tyr Ala Asn Glu His Gly Val His Gly His Trp Thr Tyr Asp Pro Glu
    50                  55                  60

Thr Lys Thr Phe Thr Val Thr Glu Gly Gly Ser Gly Gly Ser Gly Gly

65                  70                  75                  80

Ser Gly Gly Ser Gly Gly Ser Gly Gly Ser Gly Gly Ser Gly Gly Ser
                85                  90                  95

Gly Gly Ser Gly Gly Ser Gly Gly Ser Gly Gly Ser Gly Gly Ser Gly
            100                 105                 110

Gly Ser Asp Thr Tyr Lys Leu Ile Leu Asn Gly Lys Thr Leu Lys Gly
        115                 120                 125

Glu Thr Thr Thr Glu Ala Val Asp Ala Ala Thr Ala Glu Lys Val Phe
    130                 135                 140

Lys His Tyr Ala Asn Glu His Gly Val His Gly His Trp Thr Tyr Asp
145                 150                 155                 160

Pro Glu Thr Lys Thr Phe Thr Val Thr Glu Arg Pro Glu Val Ile Asp
                165                 170                 175

Ala Ser Glu Leu Thr Pro Ala Val Gly Gly Gly Gly Ser Gly Gly Gly
            180                 185                 190

Gly Ser Gly Gly Gly Gly Ser Gly Gly Gly Gly Cys His His His His
        195                 200                 205

His His
    210

<210>  43
<211>  204
<212>  PRT
<213>  Artificial Sequence

<220>
<223>  PG2LL6

<400>  43

Met Ala Tyr Tyr Asp Pro Glu Thr Gly Thr Trp Tyr Ser Gly Gly Arg
1               5                   10                  15

Asp Thr Tyr Lys Leu Ile Leu Asn Gly Lys Thr Leu Lys Gly Glu Thr
            20                  25                  30

Thr Thr Glu Ala Val Asp Ala Ala Thr Ala Glu Lys Val Phe Lys His
        35                  40                  45

Tyr Ala Asn Glu His Gly Val His Gly His Trp Thr Tyr Asp Pro Glu
    50                  55                  60

Thr Lys Thr Phe Thr Val Thr Glu Gly Gly Ser Gly Gly Ser Gly Gly
65                  70                  75                  80

Ser Gly Gly Ser Gly Gly Ser Gly Gly Ser Gly Gly Ser Gly Gly Ser
                85                  90                  95

Gly Gly Ser Gly Gly Ser Gly Gly Ser Gly Gly Ser Asp Thr Tyr Lys
            100                 105                 110

Leu Ile Leu Asn Gly Lys Thr Leu Lys Gly Glu Thr Thr Thr Glu Ala
        115                 120                 125

Val Asp Ala Ala Thr Ala Glu Lys Val Phe Lys His Tyr Ala Asn Glu
    130                 135                 140

His Gly Val His Gly His Trp Thr Tyr Asp Pro Glu Thr Lys Thr Phe
145                 150                 155                 160

Thr Val Thr Glu Arg Pro Glu Val Ile Asp Ala Ser Glu Leu Thr Pro
                165                 170                 175

Ala Val Gly Gly Gly Gly Ser Gly Gly Gly Gly Ser Gly Gly Gly Gly
            180                 185                 190

Ser Gly Gly Gly Gly Cys His His His His His His
        195                 200

<210>  44
<211>  198
<212>  PRT
<213>  Artificial Sequence

<220>
<223>  PG2LL5

<400>  44

Met Ala Tyr Tyr Asp Pro Glu Thr Gly Thr Trp Tyr Ser Gly Gly Arg
1               5                   10                  15

Asp Thr Tyr Lys Leu Ile Leu Asn Gly Lys Thr Leu Lys Gly Glu Thr
            20                  25                  30

Thr Thr Glu Ala Val Asp Ala Ala Thr Ala Glu Lys Val Phe Lys His
        35                  40                  45

Tyr Ala Asn Glu His Gly Val His Gly His Trp Thr Tyr Asp Pro Glu
    50                  55                  60

Thr Lys Thr Phe Thr Val Thr Glu Gly Gly Ser Gly Gly Ser Gly Gly
65                  70                  75                  80

Ser Gly Gly Ser Gly Gly Ser Gly Gly Ser Gly Gly Ser Gly Gly Ser
                85                  90                  95

Gly Gly Ser Gly Gly Ser Asp Thr Tyr Lys Leu Ile Leu Asn Gly Lys
            100                 105                 110

Thr Leu Lys Gly Glu Thr Thr Thr Glu Ala Val Asp Ala Ala Thr Ala
        115                 120                 125

Glu Lys Val Phe Lys His Tyr Ala Asn Glu His Gly Val His Gly His
    130                 135                 140

Trp Thr Tyr Asp Pro Glu Thr Lys Thr Phe Thr Val Thr Glu Arg Pro
145                 150                 155                 160

Glu Val Ile Asp Ala Ser Glu Leu Thr Pro Ala Val Gly Gly Gly Gly
                165                 170                 175

Ser Gly Gly Gly Gly Ser Gly Gly Gly Gly Ser Gly Gly Gly Gly Cys
            180                 185                 190

His His His His His His
        195

<210>  45
<211>  192
<212>  PRT
<213>  Artificial Sequence

<220>
<223>  PG2LL4

<400>  45

Met Ala Tyr Tyr Asp Pro Glu Thr Gly Thr Trp Tyr Ser Gly Gly Arg
1               5                   10                  15

Asp Thr Tyr Lys Leu Ile Leu Asn Gly Lys Thr Leu Lys Gly Glu Thr
            20                  25                  30

Thr Thr Glu Ala Val Asp Ala Ala Thr Ala Glu Lys Val Phe Lys His
        35                  40                  45

Tyr Ala Asn Glu His Gly Val His Gly His Trp Thr Tyr Asp Pro Glu
    50                  55                  60

Thr Lys Thr Phe Thr Val Thr Glu Gly Gly Ser Gly Gly Ser Gly Gly
65                  70                  75                  80

Ser Gly Gly Ser Gly Gly Ser Gly Gly Ser Gly Gly Ser Gly Gly Ser
                85                  90                  95

Asp Thr Tyr Lys Leu Ile Leu Asn Gly Lys Thr Leu Lys Gly Glu Thr
            100                 105                 110

Thr Thr Glu Ala Val Asp Ala Ala Thr Ala Glu Lys Val Phe Lys His
        115                 120                 125

Tyr Ala Asn Glu His Gly Val His Gly His Trp Thr Tyr Asp Pro Glu
    130                 135                 140

Thr Lys Thr Phe Thr Val Thr Glu Arg Pro Glu Val Ile Asp Ala Ser
145                 150                 155                 160

Glu Leu Thr Pro Ala Val Gly Gly Gly Gly Ser Gly Gly Gly Gly Ser
                165                 170                 175

Gly Gly Gly Gly Ser Gly Gly Gly Gly Cys His His His His His His
            180                 185                 190

<210>  46
<211>  174
<212>  PRT
<213>  Artificial Sequence

<220>
<223>  PG2LL1

<400>  46

Met Ala Tyr Tyr Asp Pro Glu Thr Gly Thr Trp Tyr Ser Gly Gly Arg
1               5                   10                  15

Asp Thr Tyr Lys Leu Ile Leu Asn Gly Lys Thr Leu Lys Gly Glu Thr
            20                  25                  30

Thr Thr Glu Ala Val Asp Ala Ala Thr Ala Glu Lys Val Phe Lys His
        35                  40                  45

Tyr Ala Asn Glu His Gly Val His Gly His Trp Thr Tyr Asp Pro Glu
    50                  55                  60

Thr Lys Thr Phe Thr Val Thr Glu Gly Gly Ser Gly Gly Ser Asp Thr
65                  70                  75                  80

Tyr Lys Leu Ile Leu Asn Gly Lys Thr Leu Lys Gly Glu Thr Thr Thr

                85                  90                  95

Glu Ala Val Asp Ala Ala Thr Ala Glu Lys Val Phe Lys His Tyr Ala
            100                 105                 110

Asn Glu His Gly Val His Gly His Trp Thr Tyr Asp Pro Glu Thr Lys
        115                 120                 125

Thr Phe Thr Val Thr Glu Arg Pro Glu Val Ile Asp Ala Ser Glu Leu
    130                 135                 140

Thr Pro Ala Val Gly Gly Gly Gly Ser Gly Gly Gly Gly Ser Gly Gly
145                 150                 155                 160

Gly Gly Ser Gly Gly Gly Gly Cys His His His His His His
                165                 170

## Description

### Technical Field

The present invention relates to protein constituted by linking plural domains having affinity for a protein comprising the Fc region of immunoglobulin G (IgG) with linkers, especially to said protein wherein an extended length to the maximum of the linker is about 80 -240 angstrom "Å. "

### Background Art

The purification of proteins such as antibodies has been an important problem for the study in biochemistry, for which various techniques are known such as affinity chromatography, gel filtration chromatography and ion-exchange chromatography. The affinity chromatography is a method for the purification of a target protein by means of a specific affinity for the target protein. Although this method makes it possible to easily and selectively collect the protein, it will be necessary in many cases to dissociate the protein absorbed to the chromate-fillers by elution with an acidic buffer of about pH2.5 due to its very strong affinity. As the decrease in activity due to denaturation of the protein will often occur under such strongly acidic conditions, a purification under milder conditions has been desired.

Protein G, which is a protein derived from streptococus, is a membrane protein present in a cell membrane of streptococcus of genus Streptococcus, and it is known that the protein G has specific binding property to the Fc region of immunoglobulin G which is a kind of the antibody (Non-patent Document 1, Patent Document 1). The protein G is a multi-domain type membrane protein consisting of a plurality of domains, some extracellular domains of which exhibit the binding property to the protein having the Fc region of immunoglobulin G (hereinafter, referred to as "antibody binding property") (Non-patent Document 2). For example, in a protein G derived from a G148 strain shown in FIG. 1, three domains of B1, B2 and B3 exhibit the antibody binding property (also written as "C1, C2 and C3 domains" depending on a document). Also, a protein G from a GX7805 strain has three antibody binding domains and a protein G from a GX7809 has two antibody binding domains. These proteins are all miniature proteins with less than 60 amino acids, and it is known that they have high identity among each amino acid sequence). It is also known that even if the protein G is cut to isolate each domain alone, the antibody binding property is maintained (Non-patent Document 3).

Many extracellular domain of protein G-containing products utilizing the selective antibody binding property of the extracellular domain of the protein G are currently marketed (for example, a carrier for affinity chromatography for purifying the antibody (Patent Documents 3 and 4), an inspection reagent and a research reagent for detecting the antibody, and the like). It is known that a binding power between the extracellular domain of the protein G and the antibody is high in a neutral to weakly acidic region and is low in a strongly acidic region (Non-patent Document 4). Therefore, when isolating, recovering and purifying the antibody, first, a sample solution containing the antibody, such as a serum, is made in a neutral state and then is brought into contact with a water-insoluble solid support, such as a bead, to which the extracellular domain of the protein G is immobilized, so that the antibody is selectively absorbed. Next, the water-insoluble solid support is washed with a neutral to weak acid solution (pH 5 to pH 8) to remove components other than the antibody. Last, a strongly acidic solution having pH 2.4 to pH 3.5 is generally added to desorb the antibody from the immobilized protein G and to elute the antibody with the strongly acidic solution (Patent Document 3). By this process, the antibody can be isolated, recovered and purified with high purity.

However, the antibody may be degraded by the strongly acidic solution having pH 2.4 to pH 3.5 due to denatured aggregation or the like, and, depending on the type of the antibody, an original function may be lost (Non-patent Document 4). Although the process is attempted in a weakly acidic region of above pH 2.4 to pH 3.5 in order to solve such a problem, the antibody is not eluted from the protein G in such weakly acidic region because the binding power between the extracellular domain of the protein G and the antibody is strong, so that a sufficient recovery amount is not attained. Moreover, it is known that the extracellular domain of the protein G also binds to an Fab region (Non-patent Document 2), and one antibody molecule can bind to the extracellular domain of the protein G in two regions, the Fc region and the Fab region. In such binding state, the antibody and the extracellular domain of the protein G cannot be easily dissociated, so that it becomes difficult to recover the antibody.

In the past, the inventors have developed an improved protein consisting of extracellular domain mutants of the protein G with thermal stability, chemical resistance to a denaturing agent, resistance to a proteolytic enzyme, and the like (these properties are also generally referred to as "protein stability" in brief) (Patent Document 5 and Patent Document 6), and have further developed an improved protein with decreased binding property to the Fc region of immunoglobulin and/or decreased binding property to the Fab region thereof in the weakly acidic region (Patent Document 7). However, each of these improved proteins contains only one domain exhibiting the antibody binding property.

Furthermore, the inventors developed protein consisting of a tandem-type multimer of said improved protein (Patent Document 8). The binding property of the protein to the Fc region of human immunoglobulin Gs included in the different subclasses such as IgGland IgG3 in the weakly acidic region is largely decreased in comparison with a tandem-type multimer of the B 1 domain of a wild-type protein G. Therefore, in the column for the chromatography for separating and purifying the protein in which a capturing agent of said protein is filled, the captured antibody can be more easily eluted without denaturation in the weakly acidic region of about pH 4-5.

It is well known that protein A that is a protein derived from Staphylococcus aureus has a specific binding property to the Fc region of immunoglobulin G (Non-patent Document 5 and Non-patent Document 6). The protein A is a multidomain-type membrane protein consisting of a plurality of domains, some extracellular domains of which exhibit the binding property to the protein having the Fc region of immunoglobulin G (hereinafter, referred to as "antibody binding property") (Non-patent Document 6). For example, in a protein A derived from a NCTC8325 strain shown in FIG. 1, five domains of E, D, A, B and C exhibit the antibody binding property. These proteins are all miniature proteins with less than 60 amino acids, and it is known that they have high identity among each amino acid sequence. It is also known that even if the protein A is cut to isolate each domain alone, the antibody binding property is maintained (Non-patent Document 7). On the other hand, a "Z" domain is an artificial protein that is synthesized based on a sequence of the B domain (Non-patent Document 7), which has two different amino acid residues from those of the B domain. It is known that said change of the two amino acid residues of "Ala1Val" to "Gly29Ala" stabilizes its structure so that its termo- denaturation temperature is 90°C or more without losing its antibody-binding property (Non-patent Document 7).

Many products utilizing the selective antibody binding property of the extracellular domain s (E, D, A, B and C) of the protein A and Z domain are currently marketed (for example, a carrier for affinity chromatography for purifying the antibody, an inspection reagent and a research reagent for detecting the antibody, and the like). It is known that a binding power between the extracellular domain of the protein A and the antibody is high in a neutral region and is low in a strongly acidic region (Non-patent Document 8). Therefore, when isolating, recovering and purifying the antibody, first, a sample solution containing the antibody, such as a serum, is made in a neutral state and then is brought into contact with a water-insoluble solid support, such as a bead, to which the extracellular domain of the protein A is immobilized, so that the antibody is selectively absorbed. Next, the water-insoluble solid support is washed with a neutral solution (pH 7) to remove components other than the antibody. Last, a strongly acidic solution having pH 3.0 is generally added to desorb the antibody from the immobilized protein A and to elute the antibody with the strongly acidic solution. By this process, the antibody can be isolated, recovered and purified with high purity.

The invenotrs have also developed a mutant protein and a capturing agent of antibodies, which comprise an extracellular domain mutant of the protein A with an improved dissociating property in the weakly acidic region (Non-patent Document 9), and a mutant-type protein and a capturing agent of antibodies, which comprise the extracellular domain mutant of the protein A with a decreased affinity in the acidic region (Non-patent Document 10)

### [Prior Arts]

### [Patent Documents]

[Patent Document 1] JP 03-501801 W
[Patent Document 2] Japanese Patent No.2764021
[Patent Document 3] JP 03-128400 A
[Patent Document 4] JP 2003-088381 A
[Patent Document 5] JP 2009-95322 A
[Patent Document 6] JP 2009-118749 A
[Patent Document 7] JP 2009-297018 A
[Patent Document 8] WO 2013/018880 A
[Patent Document 9] JP 2010-81866 A
[Patent Document 10] WO2012/165544 A

### [Non-patent Documents]

[Non-patent Document 1] Bjorck L, Kronvall G. (1984) Purification and some properties of streptococcal protein G, a novel IgG-binding reagent. J Immunol. 133, 969-974.
[Non-patent Document 2] Boyle M. D.P., Ed. (1990) Bacterial Immunoglobulin Binding Proteins. Academic Press, Inc., San Diego, CA, USA.
[Non-patent Document 3] Gallagher T, Alexander P, Bryan P, Gilliland GL. (1994) Two crystal structures of the B 1 immunoglobulin-binding domain of streptococcal protein G and comparison with NMR. Biochemistry 19, 4721-4729.
[Non-patent Document 4] Gagnon P. (1996) Purification Tools for Monoclonal Antibodies, Validated Biosystems Inc., Tucson, AZ, USA.
[Non-patent Document 5] Forsgren A and Sjoquist J (1966) "Protein A" from S. Aureus. J Immunol. 97, 822-827.
[Non-patent Document 6] Boyle M. D.P., Ed. (1990) Bacterial Immunoglobulin Binding Proteins. Academic Press, Inc., San Diego, CA, USA.
[Non-patent Document 7] Tashiro M, Montelione GT. (1995) Structures of bacterial immunoglobulin-bindingdomains and their complexes with immunoglobulins. Curr Opin Struct Biol. 5, 471-481.
[Non-patent Document 8] Gagnon P. (1996) Purification Tools for Monoclonal Antibodies, Validated Biosystems Inc., Tucson, AZ, USA.
[Non-patent Document 9] Evers TH, van Dongen EM, Faesen AC, Meijer EW, Merkx M., (2006) Quantitative understanding of the energy transfer between fluorescent proteins connected via flexible peptide linkers, Biochemistry. Nov 7;45(44):13183-92

### Summary of Invention

### Problems to be Solved by the Invention

Therefore, the technical problem solved by the present invention is to provide a novel protein, which have such excellent pH responsiveness that the antibody binding property to the Fc region of the immunoglobulin in the neutral region is improved and the binding property to the Fc region of the immunoglobulin in the weakly acidic region is much decreased when compared to the wild-type proteins such as protein G and protein A that have affinity for the protein comprising the Fc region of the immunoglobulin G (IgG), and the mutant-type protein (an improved protein) comprising their domain mutants.

Furthermore, the technical problem solved by the present invention is to provide a capturing agent for the antibody, the immunoglobulin G or the protein having the Fab region of immunoglobulin G (such as the antibody), which is characterized in that the former protein is immobilized, and is useful as a filler for an affinity chromatography for purifying the antibody; and a column for the chromatography for separating and purifying the proteinthat is filled with said capturing agent, especially a column for the affinity chromatography for purifying the antibody.

The inventors then found that the above problems have been resolved by adjusting the length of linkers that link each of the domains in said wild-type proteins and their mutant-typw proteins in an appropriate range, and leading to the completion of the present invention.

Namely, each aspect of the present invention is as follows.

### Aspect 1

A protein constituted by linking plural domains having affinity for a protein comprising the Fc region of the immunoglobulin G (IgG) with linkers wherein an extended length to the maximum of the linker is 80 angstrom -240 angstrom.

### Aspect 2

The protein according to aspect 1 wherein the linker is a polypeptide linker.

### Aspect 3

The protein according to aspect 2 wherein the linker consists of a polypeptide of 22-66 amino acid residues.

### Aspect 4

The protein according to aspect 3 wherein the amino acid sequence of the linker consists of 4 to 10 times repeats of GlyGlySerGlyGlySer.

### Aspect 5

The protein according to aspect 4 wherein the amino acid sequence of the linker consists of 6 to 10 times repeats of GlyGlySerGlyGlySer.

### Aspect 6

The protein according to any one of aspects 1 to 5, wherein the domain is derived from protein G or protein A.

### Aspect 7

The protein according to aspect 6, wherein the domain is a mutant of any one of B1, B2 and B3 domains of a wild-type protein G.

### Aspect 8

The protein according to any one of aspects 1 to 7, wherein the domains are the same as one another.

### Aspect 9

The protein according to any one of aspects 1 to 8, which is a dimer consisting of the linked two domains.

### Aspect 10

The protein according to any one of the aspects 7 to 9, wherein the domain mutant is a mutant protein of B1 domain protein of the wild-type protein G,
the mutant protein consists of an amino acid sequence represented by (a) or of the amino acid sequence obtained by deleting, substituting, inserting or adding one or several amino acid residues in the amino acid sequence represented by (a),
the mutant protein has the binding property to the Fc region of immunoglobulin G, and
the mutant protein has at least the binding property to the Fab region of immunoglobulin G and/or the binding property to the Fc region of immunoglobulin G in the weakly acidic region is decreased in comparison with a B 1 domain protein of the wild-type protein G.

(a)AspThrTyrLysLeuIleLeuAsnGlyLysX11LeuLysGlyGluThrX17ThrGluAlaValX22AlaAla
X25AlaGluLysValPheLysX32TyrAlaX35X36X37GlyValX40GlyX42TrpThrTyrAspX47X48ThrLys
ThrPheThrValThrGlu

(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or Leu; X47 is Asp or Pro; X48 is Ala, Lys or Glu; X22 is Asp; X25 is Thr; X32 is Gln; X40 is Asp; X42 is Glu; and X11 is Thr, and X17 is Thr simultaneously.)

### Aspect 11

The protein according to any one of the aspects 7 to 9, wherein the domain mutant is a mutant protein of B2 domain protein of the wild-type protein G,
the mutant protein consists of an amino acid sequence represented by (b) or of the amino acid sequence obtained by deleting, substituting, inserting or adding one or several amino acid residues in the amino acid sequence represented by (b),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B2 domain protein of the wild-type protein G.

(b)ThrThrTyrLysLeuValIleAsnGlyLysX11LeuLysGlyGluThrX17ThrGluAlaValX22AlaAlaX
25AlaGluLysValPheLysX32TyrAlaX35X36X37GlyValX40GlyX42TrpThrTyrAspX47X48ThrLysT
hrPheThrValThrGlu

(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; X42 is Glu; and X11 is Thr and X17 is Thr simultaneously.)

### Aspect 12

The protein according to any one of the aspects 7 to 9, wherein the domain mutant is a mutant protein of B3 domain protein of the wild-type protein G,
the mutant protein consists of an amino acid sequence represented by (c) or of the amino acid sequence obtained by deleting, substituting, inserting or adding one or several amino acid residues in the amino acid sequence represented by (c),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B3 domain protein of the wild-type protein G.

(c)ThrThrTyrLysLeuValIleAsnGlyLysX11LeuLysGlyGluThrX17ThrLysAlaValX22AlaGluX
25AlaGluLysAlaPheLysX32TyrAlaX35X36X37GlyValX40GlyValTrpThrTyrAspX47X48ThrLysTh
rPheThrValThrGlu

(In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; and X11 is Thr and X17 is Thr simultaneously.)

### Aspect 13

The protein according to any one of the aspects 7 to 9, wherein the domain mutant is a mutant protein of B 1 domain protein of the wild-type protein G,
the mutant protein consists of an amino acid sequence represented by (d) or of the amino acid sequence obtained by deleting, substituting, inserting or adding one or several amino acid residues in the amino acid sequence represented by (d),
the mutant protein has the binding property to the Fc region of immunoglobulin G, and
the mutant protein has the binding property to the Fab region of immunoglobulin G and/or the binding property to the Fc region in the weakly acidic region is decreased in comparison with B 1 domain protein of the wild-type protein G.

(d)AspThrTyrLysLeuIleLeuAsnGlyLysX11LeuLysGlyGluThrX17ThrGluAlaValX22AlaAla
X25AlaGluLysValPheLysX32TyrAlaAsnAspAsnGlyValX40GlyX42TrpThrTyrAspAspAlaThrLysT
hrPheThrValThrGlu

(In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

### Aspect 14

The protein according to any one of the aspects 7 to 9, wherein the domain mutant is a mutant protein of B2 domain protein of the wild-type protein G,
the mutant protein consists of an amino acid sequence represented by (e) or of the amino acid sequence obtained by deleting, substituting, inserting or adding one or several amino acid residues in the amino acid sequence represented by (e),
the mutant protein has the binding property to the Fc region of immunoglobulin G, and
the mutant protein has the binding property to the Fab region of immunoglobulin G and/or the binding property to the Fc region in the weakly acidic region is decreased in comparison with B2 domain protein of the wild-type protein G.

(e)ThrThrTyrLysLeuValIleAsnGlyLysX11LeuLysGlyGluThrX17ThrGluAlaValX22AlaAlaX
25AlaGluLysValPheLysX32TyrAlaAsnAspAsnGlyValX40GlyX42TrpThrTyrAspAspAlaThrLysThr
PheThrValThrGlu

(In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

### Aspect 15

The protein according to any one of the aspects 7 to 9, wherein the domain mutant is a mutant protein of B3 domain protein of the wild-type protein G,
the mutant protein consists of an amino acid sequence represented by (f) or of the amino acid sequence obtained by deleting, substituting, inserting or adding one or several amino acid residues in the amino acid sequence represented by (f),
the mutant protein has the binding property to the Fc region of immunoglobulin G, and
the mutant protein has the binding property to the Fab region of immunoglobulin G and/or the binding property to the Fc region in the weakly acidic region is decreased in comparison with B3 domain protein of the wild-type protein G.

(f)ThrThrTyrLysLeuValIleAsnGlyLysX11LeuLysGlyGluThrX17ThrLysAlaValX22AlaGluX
25AlaGluLysAlaPheLysX32TyrAlaAsnAspAsnGlyValX40GlyValTrpThrTyrAspAspAlaThrLysThr
PheThrValThrGlu

(In the amino acid sequence, X22 represents Asp or His; X25 represents Thr or His; X32 represents Gln or His; X40 represents Asp or His; X11 represents Thr or Arg; and X17 represents Thr or Ile, respectively, with the proviso that a case is excluded where X22 is Asp; X25 is Thr; X32 is Gln; X40 is Asp; and X11 is Thr and X17 is Thr simultaneously.)

### Aspect 16

The protein according to any one of the aspects 7 to 9, wherein the domain mutant is a mutant protein of B 1 domain protein of the wild-type protein G,
the mutant protein consists of an amino acid sequence represented by (g) or of the amino acid sequence obtained by deleting, substituting, inserting or adding one or several amino acid residues in the amino acid sequence represented by (g),
the mutant protein has the binding property to the Fc region of immunoglobulin G, and
the mutant protein has the binding property to the Fc region in the weakly acidic region is decreased in comparison with B 1 domain protein of the wild-type protein G.

(g)AspThrTyrLysLeuIleLeuAsnGlyLysThrLeuLysGlyGluThrThrThrGluAlaValX22AlaAlaX
25AlaGluLysValPheLysX32TyrAlaAsnAspAsnGlyValX40GlyX42TrpThrTyrAspAspAlaThrLysThr
PheThrValThrGlu

(In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

### Aspect 17

The protein according to any one of the aspects 7 to 9, wherein the domain mutant is a mutant protein of B2 domain protein of the wild-type protein G,
the mutant protein consists of an amino acid sequence represented by (h) or of the amino acid sequence obtained by deleting, substituting, inserting or adding one or several amino acid residues in the amino acid sequence represented by (h),
the mutant protein has the binding property to the Fc region of immunoglobulin G, and
the mutant protein has the binding property to the Fc region in the weakly acidic region is decreased in comparison with B2 domain protein of the wild-type protein G.

(h)ThrThrTyrLysLeuValIleAsnGlyLysThrLeuLysGlyGluThrThrThrGluAlaValX22AlaAlaX2

5AlaGluLysValPheLysX32TyrAlaAsnAspAsnGlyValX40GlyX42TrpThrTyrAspAspAlaThrLysThrP
heThrValThrGlu

(In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

### Aspect 18

The protein according to any one of the aspects 7 to 9, wherein the domain mutant is a mutant protein of B3 domain protein of the wild-type protein G,
the mutant protein consists of an amino acid sequence represented by (i) or of the amino acid sequence obtained by deleting, substituting, inserting or adding one or several amino acid residues in the amino acid sequence represented by (i),
the mutant protein has the binding property to the Fc region of immunoglobulin G, and
the mutant protein has the binding property to the Fc region in the weakly acidic region is decreased in comparison with B3 domain protein of the wild-type protein G.

(i)ThrThrTyrLysLeuValIleAsnGlyLysThrLeuLysGlyGluThrThrThrLysAlaValX22AlaGluX2
5AlaGluLysAlaPheLysX32TyrAlaAsnAspAsnGlyValX40GlyValTrpThrTyrAspAspAlaThrLysThrP
heThrValThrGlu

(In the amino acid sequence, X22 represents Asp or His; X25 represents Thr or His; X32 represents Gln or His; and X40 represents Asp or His, respectively, with the proviso that a case is excluded where X22 is Asp; and X25 is Thr; X32 is Gln and X40 is Asp simultaneously.)

### Aspect 19

The protein according to any one of the aspects 7 to 9, wherein the domain mutant is a mutant protein consists of an amino acid sequence represented by (j):

(j)AspThrTyrLysLeuIleLeuAsnGlyLysThrLeuLysGlyGluThrThrThrGluAlaValAspAlaAlaTh
rAlaGluLysValPheLysHisTyrAlaAsnGluHisGlyValHisGlyHisTrpThrTyrAspProGluThrLysThrPheT

hrValThrGlu.

### Aspect 20

A protein that is a fusion protein consisting of a linked amino acid sequence of an amino acid sequence of the protein according to any one of the aspects 1 to 19 with an amino acid sequence of another protein.

### Aspect 21

The protein according to aspect 20, having an amino acid sequence represented by any one of SEQ ID NO. 41 to 45.

### Aspect 22

A nucleic acid encoding the protein according to any one of the aspects 1 to 21.

### Aspect 23

A recombinant vector comprising the nucleic acid according to aspect 22.

### Aspect 24

A transformant into which the recombinant vector according to aspect 23 is introduced.

### Aspect 25

A capturing agent for immunoglobulin G, or protein comprising the Fc or Fab region of the immunoglobulin G, wherein the protein according to any one of the aspects 1 to 21 is immobilized to a water-insoluble solid support.

### Aspect 26

An affinity chromatography comprising the capturing agent according to aspect 25 for the purification of immunoglobulin G, or the protein comprising the Fc or Fab region of the immunoglobulin G.

### Aspect 27

A method for the purification of immunoglobulin G, or the protein comprising the Fc or Fab region of the immunoglobulin G by means of the affinity chromatography for the purification according to aspect 26.

### Advantages of Invention

According to the present invention, it was found that by adjusting an extended length to the maximum of the linker that links the plural domains having affinity (specific binding property) for the protein comprising the Fc region of the immunoglobulin G (IgG) to a range of 80 -240 angstrom, such excellent pH responsiveness was shown that the antibody binding property to the Fc region of the immunoglobulin in the neutral region is remarkably improved and the binding property to the Fc region of the immunoglobulin in the weakly acidic region is much decreased when compared to the wild-type proteins such as protein G and protein A wherein peptides having about 22-66 amino acids are comprised between each of their domains, and to the prior mutant-type protein (the improved-type protein) comprising their domain mutants.

Surprisingly, it has become possible to dissociate and elute the immunoglobulin under much milder conditions in the weakly acidic region while maintaining the antibody binding property to the immunoglobulin such as human IgG in the neutral region by utilizing the protein according to the present invention as the capturing agent. As a result, it isnow possible to recover the immunoglobulin, which is liable to denaturation with an acid, at pH around the neutral region in the affinity purification without any risk of its denaturation due to the acid.

### Brief Description of Drawings

FIG. 1 shows the structures of the tandem-type dimers of extracellular domain mutants of the protein G (PG2LL10, PG2LL7, PG2LL6, PG2LL5, PG2LL4, PG2LL1).
FIG. 2-1 shows the results of the SPR measurement under the protein immobilization condition in which proteins of the same mass are immobilized, the results showing the comparison between the tandem-type dimers of extracellular domain mutants of the protein G (PG2LL5, PG2LL4, PG2LL1).
FIG. 2-2 shows the results of the SPR measurement under the protein immobilization condition in which proteins of the same mass are immobilized, the results showing the comparison between the tandem-type dimers of extracellular domain mutants of the protein G (PG2LL10, PG2LL7, PG2LL6).
FIG. 3 is a bar graph showing the comparison betweena binding rate of the tandem-type dimers of extracellular domain mutants of the protein G (PG2LL10, PG2LL7, PG2LL6, PG2LL5, PG2LL4, PG2LL1). Relative binding stability (%) was calculated from the SPR data of PG2LL4-6 provided that SPR response of PGLL1 and PGLL10 are 0% and 100%, respectively, in 500 sec after the completion of the antibody addition.
FIG. 4 shows results of the pH gradient affinity chromatography in the present recombinant portein G-immobilized columns.

### Embodiments of the Invention

The present invention relates to the protein constituted by linking with linkers the plural domains having affinity for the protein comprising the Fc region of immunoglobulin G (IgG), which is characterized in that an extended length to the maximum of the linker is adjusted in a range of about 80-240 angstrom Å."

Although there is no specific limitation on the chemical structure of the linkers comprised in the protein according to the present invention, a (poly)peptide linker consisting of amino acids is preferable. There is no limitation on the kind and sequence of the amino acids constituting the polypeptide linker. For example, it may consist of repeated units of a peptide having plural amino acids. A preferable example of the repeated unit is "GlyGlySerGlyGlySer." This sequence is generally used in designing protein such as a single-chain antibody in the art, and has advantageous properties such as a low immunogenicity causing little influence on living bodies.

As the extended length to the maximum of the above unit of "GlyGlySerGlyGlySer" is about 21.6 angstrom, calculated on the description on page 34 of Harper's Biochemistry, MARUZEN, a peptide linker consisting of 4 to 11 times repeats of the unit of GlyGlySerGlyGlySer (24-66 amino acids in total) will have the extended length to the maximum of about 86-240 angstrom, and a peptide linker consisting of 4 to 10 times repeats of the same unit (24-60 amino acids in total) will have the extended length to the maximum of about 86-216 angstrom.

Accordingly, the number of the amino acids constituting the peptide linkers comprised in the protein of the present invention is usually about 22-26, preferably about 24-60, depending on the kind of the amino acids (their chemical structure). For example, the peptide linker is preferably 4 to 10 times repeats of the unit of GlyGlySerGlyGlySer, more preferably 6 to 10 times repeats of the same unit.

As there is no limitation on the kind, structure or origin of the domain comprised in the protein according to the present invention, and any known one may be used as long as it has the affinity for the protein comprising the Fc region of immunoglobulin G (IgG). Preferable examples include the domain derived from protein G or protein A, such as their wild-type domain, and their domain mutant wherein a part of the amino acid sequence of the wild-type domain are changed, such as a mutant of any one of B1, B2 and B3 domains of the wild-type protein G. Preferable examples of such domains are more specifically described in the present specification. The mutation of the amino acids in such improved protein may be carried out by any method known to those skilled in the art such as, for example, the method described in examples of Patent Document 8.

The number of the domains comprised in the protein according to the present invention may be optionally selected depending on the use of the protein and the kinds of the domains. The protein can be a dimer, a trimer, a tetramer or a pentamer. The domains comprised in the protein of the present invention is different from one another or the same as one another.

The distance between the two domains of the protein G in the complex wherein the bivalent Fc region of the antibody interacts with the protein G was estimated as about 66 angstrom from the calculation based on the known structure information (Sauer-Eriksson AE, Kleywegt GJ, Uhlen M, Jones TA. Crystal structure of the C2 fragment of streptococcal protein G in complex with the Fc domain of human IgG. Structure. 1995 Mar 15;3(3):265-78) using a structure-drawing software (for example, ViewerLite 5.0 (Accelrys Inc)). Accordingly, a linker with the length of about 66 angstrom or more will be necessary if the two domains are involved in the interaction with bivalency. However, since the linker comprised in the wild-type protein G has 14 amino acid residues, the extended length to the maximum of said linker cannot be more than about 50 angstrom. A sufficient length may be obtained by increasing the number of the domains comprised in the protein. However, increase in the number of the domain would make the protein structure more complex, causing more often such problems that precipitation would occur more easily in a solution, and dispersion of the protein in carriers would be deteriorated due to the increase volume of the protein.

On the other hand, in the protein according to the present invention, since the extended length to the maximum of the linker is adjusted in a range of about 80 -240 angstrom, even the dimer having two domains can interact with the bivalency of the Fc region of the antibody without any spatial difficulty, causing no such problems as above.

The immunoglobulin G may include various kinds of antibodies of human, and of other animals than human, especially of mammalians such as rat, mice, hamster, goat and rabbit, and various fragments of each antibody such as Fab fragment of the human IgG. There is no limitation in their structure or constituents as long as they comprise Fc or Fab region, being any one of various- types of the antibodies and their fragments known for those skilled in the art. Thus, there may be mentioned a single-chain antibody (scFv), a dimer of the scFv, a diabody-type bispecific antibody, and a multimer of low-molecular antibodies, and various antibody fragments such as Fab fragment, F(ab')₂ and Fab'in addition to a normal (intact) IgG-type antibody molecule.

As preferable aspects of the domain mutants comprised in the protein of the present invention, mutant proteins are listed below.

A. The first aspect of the mutant proteins of the present invention is described in the following (1) (2).
(1) A mutant protein prepared by substituting another amino acid residue for any one or more of amino acid residues: Asp22, Ala24, Thr25, Lys28, Val29, Lys31, Gln32, Asn35, Asp36, Gly38, Asp40, Glu42, Thr44, as a target part for the mutation in a B1 or B2 domain protein of a wild-type protein G consisting of an amino acid sequence represented by SEQ ID NO. 1 or 2, characterized in that an amino acid residue described in any one of the following (i) to (iii) is substituted for each amino acid residue as the target part for the mutation, wherein the mutant protein has the binding property to the Fc region of immunoglobulin G, and wherein the binding property of the mutant protein to the Fc region of immunoglobulin G in the weakly acidic region is decreased in comparison with the B 1 or B2 domain protein of the wild-type protein G.
   (i) Substitution to a charged amino acid residue when the amino acid residue as the target part for the mutation is an uncharged amino acid residue.
   (ii) Substitution to a charged amino acid residue with opposite electric charge when the amino acid residue as the target part for the mutation is a charged amino acid residue.
   (iii) Substitution to a histidine residue for the amino acid residue as the target part for the mutation.
(2) A mutant protein prepared by substituting another amino acid residue for any one or more of amino acid residues: Asp22, Thr25, Lys28, Lys31, Gln32, Asn35, Asp36, Gly38, Asp40, Thr44, as a target part for the mutation in a B3 domain protein of a wild-type protein G consisting of an amino acid sequence represented by SEQ ID NO. 3, characterized in that an amino acid residue described in any one of the following (i) to (iii) is substituted for each amino acid residue as the target part for the mutation, wherein the mutant protein has the binding property to the Fc region of immunoglobulin G, and wherein the binding property of the mutant protein to the Fc region of immunoglobulin G in the weakly acidic region is decreased in comparison with the B3 domain protein of the wild-type protein G.
   (i) Substitution to a charged amino acid residue when the amino acid residue as the target part for the mutation is an uncharged amino acid residue.
   (ii) Substitution to a charged amino acid residue with opposite electric charge when the amino acid residue as the target part for the mutation is a charged amino acid residue.
   (iii) Substitution to a histidine residue for the amino acid residue as the target part for the mutation.

The above-mentioned mutant proteins described in (1) and (2) are designed based on a target part for the mutation selected as follows and the amino acid residue which is substituted for the target part, and are obtained by a genetic engineering technique.

### [Selection of the target part for the mutation and specification of the amino acid residue which is substituted, based on an analysis of a surface bound to the Fc]

The part where the mutation is transduced for designing the amino acid sequence of the mutant protein of the present invention is selected by using a three-dimensional atomic coordinate data (Reference Document 4) of a complex in which the B2 domain of the protein G and the Fc region of immunoglobulin G are bound together. To decrease the antibody-binding property of the extracellular domain of the protein G in the weakly acidic region, substitution for an amino acid residue in a binding surface of the extracellular domain of the protein G directly related to the binding to the Fc region and substitution for surrounding amino acid residues thereof should be executed from the wild-type to a non wild-type. Therefore, first, in the complex in which the B2 domain of the protein G and the Fc region of immunoglobulin G had been bound together, amino acid residues of the B2 domain of the protein G within a fixed distance range from the Fc region were specified, and were selected as candidates of the target part for the mutation. Next, to minimize structural destabilization of the extracellular domain of the protein G associated with the amino acid substitution, among the above-mentioned candidates, only amino acid residues of the B2 domain of the protein G which had been exposed on a molecular surface were determined as the target parts for the mutation.

Thus, specifically, as shown in the following examples, by setting the above-mentioned distance range to 6.5 angstroms or less and exposed surface area ratio to 40 % and over, thirteen amino acid residues of Asp22, Ala24, Thr25, Lys28, Val29, Lys31, Gln32, Asn35, Asp36, Gly38, Asp40, Glu42 and Thr44 were selected as the target part for the mutation in the wild-type amino acid sequence of the B2 domain of the protein G (SEQ ID NO. 2).

Moreover, as described above, since there exists extremely high sequence identity among each extracellular domain of the protein G and little difference among each stereoscopic structure of the B1. B2 and B3 domains, the finding on the stereoscopic structure of the B2 domain - Fc complex is applicable to a B1 domain - Fc complex and a B3 domain - Fc complex. Therefore, not only in the B2 domain but also in the B 1 domain and the B3 domain, the thirteen amino acid residues as the target part for the mutation resulting from the stereoscopic structure of the B2 domain - Fc complex can be selected as the target part for the mutation, as long as the same kind of amino acid is in a corresponding position. Namely, thirteen amino acid residues of Asp22, Ala24, Thr25, Lys28, Val29, Lys31, Gln32, Asn35, Asp36, Gly38, Asp40, Glu42 and Thr44, in the wild-type amino acid sequence of the B 1 domain of the protein G (SEQ ID NO. 1), and ten amino acid residues of Asp22, Thr25, Lys28, Lys31, Gln32, Asn35, Asp36, Gly38, Asp40 and Thr44, in the wild-type amino acid sequence of the B3 domain of the protein G (SEQ ID NO. 3), were selected as the target part for the mutation.

On the other hand, as the amino acid residue which was substituted for the original amino acid residue as the target part for the mutation, any one of the following (i) to (iii) was specified.
(i) When the wild-type amino acid residue as the target part for the mutation is an amino acid with an uncharged side-chain (Gly, Ala, Val, Leu, Ile, Ser, Thr, Asn, Gln, Phe, Tyr, Trp, Met, Cys, Pro), an amino acid with a charged side-chain (Asp, Glu, Lys, Arg, His) is substituted. Since a chemical state of a charged amino acid is greatly changed depending on pH, the charged amino acid can cause to change the antibody-binding property of the B2 domain of the protein G in the neutral region and the weakly acidic region.
(ii) When the wild-type amino acid residue as the target part for the mutation is a charged amino acid, a charged amino acid with opposite electric charge is substituted. Similarly to above, since the chemical state of the charged amino acid is greatly changed depending on pH, the charged amino acid can cause to change the antibody-binding property of the B2 domain of the protein G in the neutral region and the weakly acidic region.
(iii) When the wild-type amino acid residue as the target part for the mutation is other than a histidine, the histidine is substituted. Since a chemical state of the histidine is greatly changed in the neutral region and the weakly acidic region, the histidine can cause to change the antibody-binding property of the B2 domain of the protein G in the neutral region and the weakly acidic region.

Specifically, as shown in the following examples, Lys, Arg or His for Asp22; Asp, Glu, Lys, Arg or His for Ala24 (only in the B 1, B2 domains); Asp, Glu, Lys, Arg or His for Thr25; Asp, Glu or His for Lys28; Asp, Glu, Lys, Arg or His for Val29 (only in the B1, B2 domains); Asp, Glu or His for Lys31; Asp, Glu, Lys, Arg or His for Gln32; Asp, Glu, Lys, Arg or His for Asn35; Lys, Arg or His for Asp36; Asp, Glu, Lys, Arg or His for Gly38; Lys, Arg or His for Asp40; Lys, Arg or His for Glu42 (only in the B1, B2 domains); and Asp, Glu, Lys, Arg or His for Thr44 were specified as the amino acid residue in positions where the substitution is executed. However, a case is excluded where an amino acid sequence according to the specification of these amino acid residues is that in which Lys is substituted for Asn35 and/or Glu is substituted for Asp36 and in which an amino acid sequence except the positions where the substitution is executed is the same as an amino acid sequence of each cell membrane domain of the wild-type protein G. Consequently, the first aspect of the mutant proteins is distinguished from the following mutant protein with the improved stability of the cell membrane domain of the protein G claimed by the inventors.

B. The second aspect of the mutant proteins of the present invention is described in the following (3).
(3) A mutant protein characterized by being prepared by substituting other kinds of amino acid residue other than a cysteine for any one or more of amino acid residues: Lys10, Thr11, Lys13, Gly14, Glu15, Thr16, Thr17, Asn35, Asp36, Gly38, as a target part for the mutation in the B1, B2 or B3 domain protein of the wild-type protein G consisting of the amino acid sequence represented by any one of SEQ ID NO. 1 to 3, wherein the mutant protein has the binding property to the Fc region of immunoglobulin G, and wherein the binding property of the mutant protein to the Fab region of immunoglobulin G is decreased in comparison with each corresponding B1, B2 or B3 domain protein of the wild-type protein G. The above-mentioned mutant protein described in (3) is designed based on a target part for the mutation selected as follows and an amino acid residue which is substituted for the target part, and is obtained by a genetic engineering technique.

### [Selection of the target part for the mutation and specification of the amino acid residue which is substituted, based on an analysis of a surface bound to the Fab]

The part where the mutation is transduced for designing the amino acid sequence of the mutant protein of the present invention is selected by using a three-dimensional atomic coordinate data (Reference Document 5) of a complex in which the B3 domain of the protein G and the Fab region of immunoglobulin G are bound together. It is known that the extracellular domain of the protein G binds to both the Fc region and the Fab region of immunoglobulin G (Reference Document 2). Therefore, one antibody molecule can simultaneously bind to the extracellular domain of a plurality of proteins G, and, in such a state, since interaction between the antibody and the extracellular domain of the proteins G is multivalent, it cannot be cut easily. Thus, to decrease the antibody-binding property of the extracellular domain of the protein G in the weakly acidic region, substitution for an amino acid residue in a binding surface of the extracellular domain of the protein G directly related to the binding to the Fab region should be executed from the wild-type to the non wild-type. Hence, first, in the complex in which the B3 domain of the protein G and the Fab region of immunoglobulin G had been bound together, amino acid residues of each cell membrane B3 domain of the protein G within a fixed distance range from the Fab region were specified, and were selected as candidates of the target part for the mutation. Next, to minimize the structural destabilization of the extracellular domain of the protein G associated with the amino acid substitution, among the above-mentioned candidates, only amino acid residues of the B3 domain of the protein G which had been exposed on the molecular surface were determined as the target parts for the mutation.

Specifically, as shown in the following examples, by setting the above-mentioned distance range to 4.0 angstroms or less and exposed surface area ratio to 40 % and over, ten amino acid residues of Lys10, Thr11, Lys13, Gly14, Glu15, Thr16, Thr17, Asn35, Asp36 and Gly38 were selected as the target part for the mutation in the wild-type amino acid sequence of the B3 domain of the protein G represented by [SEQ ID NO. 3]. Moreover, as described above, since there exists extremely high identity among each extracellular domain of the protein G, each of the B1, B2 and B3 domains has the target parts for the mutation in common. Therefore, not only in the B3 domain but also in the B 1 domain and the B2 domain, the ten amino acid residues can be selected as the target parts for the mutation.

On the other hand, the amino acid residue which is substituted for the original amino acid residue as the target part for the mutation can be specified by the following method. (iv) Other kinds of amino acid residue other than the wild-type amino acid and the cysteine is substituted. Consequently, eliminating a risk of a crosslinking reaction by the transduction of the cysteine, the decrease in the binding property with the Fab region due to the mutation of the wild-type amino acid can be produced.

Specifically, as seen in the examples below, amino acid residues other than Lys and Cys for Lys10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38; were specified as the amino acid residues which would be substituted for each extracellular domain protein of the wild-type protein G. However, a case is excluded where an amino acid sequence according to the above-mentioned selection of the amino acid residues is that in which Lys is substituted for Asn35 and/or Glu is substituted for Asp36 and in which an amino acid sequence except the positions where the substitution is executed is the same as the amino acid sequence of each cell membrane domain of the wild-type protein G. Consequently, the first aspect of the mutant proteins is distinguished from the following mutant protein with the improved stability of the cell membrane domain of the protein G claimed by the inventors.

C. The third aspect of the mutant proteins of the present invention comprises both the above-mentioned substitution of the amino acid residue for improving the binding property to the Fc region of immunoglobulin and the above-mentioned substitution of the amino acid residue for improving the binding property to the Fab region.

### [Selection of the target part for the mutation and specification of the amino acid residue which is substituted, based on the analyses of the surfaces bound to the Fc and the Fab]

By combining the above-mentioned amino acid residue which is specified based on the analysis of the surface bound to the Fc and is substituted for the target part for the mutation and the above-mentioned amino acid residue which is specified based on the analysis of the surface bound to the Fab and is substituted for the target part for the mutation, the selection of the target part for the mutation and the specification of the amino acid residue which is substituted are performed. Specifically, twenty amino acid residues of Asp22, Ala24, Thr25, Lys28, Val29, Lys31, Gln32, Asp40, Glu42, Thr44, Lys10, Thr11, Lys13, Gly14, Glu15, Thr16, Thr17, Asn35, Asp36 and Gly38 in each wild-type amino acid sequence of the B 1 and B2 domains of the protein G (SEQ ID NO. 1, 2) are selected as the target parts for the mutation. Among the target parts for the mutation, Asp22, Ala24, Thr25, Lys28, Val29, Lys31, Gln32, Asp40, Glu42 and Thr44 are target parts for improving the binding property to the Fc region, and Lys10, Thr11, Lys13, Gly14, Glu15, Thr16 and Thr17 are target parts for improving the binding property to the Fab region. Asn35, Asp36 and Gly38 are parts for improving the binding property to the Fc region as well as parts for improving the binding property to the Fab region. Therefore, the substitution to the amino acid residue for Asn35, Asp36 or Gly38 for improving the binding property to the Fc region described in A is also the substitution to another amino acid residue other than the cysteine residue for simultaneously improving the binding property to the Fab region.

Namely, as used herein, "comprising both" the substitutions of the amino acid residue is defined as not only the case that the substitutions of the amino acid residue are combined when the target part for the mutation for improving the binding property to the Fc region and the target part for the mutation for improving the binding property to the Fab region are differently selected, but also the case that the substitution of the amino acid residue described in A is executed after the same part is selected as the target part for the mutation for improving the binding property to both the regions.

Lys, Arg or His for Asp22; Asp, Glu, Lys, Arg or His for Ala24; Asp, Glu, Lys, Arg or His for Thr25; Asp, Glu, or His for Lys28; Asp, Glu, Lys, Arg or His for Val29; Asp, Glu or His for Lys31; Asp, Glu, Lys, Arg or His for Gln32; Lys, Arg or His for Asp40; Lys, Arg or His for Glu42; Asp, Glu, Lys, Arg or His for Thr44; amino acid residues other than Lys and Cys for Lys 10; amino acid residues other than Thr and Cys for Thr11; amino acid residues other than Lys and Cys for Lys13; amino acid residues other than Gly and Cys for Gly14; amino acid residues other than Glu and Cys for Glu15; amino acid residues other than Thr and Cys for Thr16; amino acid residues other than Thr and Cys for Thr17; amino acid residues other than Asn and Cys for Asn35; amino acid residues other than Asp and Cys for Asp36; and amino acid residues other than Gly and Cys for Gly38 are selected as the amino acid residues which would be substituted.

On the other hand, in the B3 domain of the protein G, seventeen amino acid residues of Asp22, Thr25, Lys28, Lys31, Gln32, Asp40, Thr44, Lys10, Thr11, Lys13, Gly14, Glu15, Thr16, Thr17, Asn35, Asp36 and Gly38 in the wild-type amino acid sequence (SEQ ID NO. 3) are selected as the target parts for the mutation. Among the amino acid residues, Asp22, Thr25, Lys28, Lys31, Gln32, Asp40 and Thr44 are target parts for the mutation for improving the binding property to the Fc region, and Lys10, Thr11, Lys13, Gly14, Glu15, Thr16 and Thr17 are target parts for the mutation for improving the binding property to the Fab region. Also, the amino acid residues have a commonality in that Asn35, Asp36 and Gly38 are the parts for improving the binding property to the Fc region as well as the parts for improving the binding property to the Fab region, so that, similar to the above-mentioned B1 or B2 domain of the protein G, the substitution to the amino acid residue for Asn35, Asp36 or Gly38 for improving the binding property to the Fc region described in A is also the substitution to another amino acid residue other than the cysteine residue for simultaneously improving the binding property to the Fab region.

However, a case is excluded where an amino acid sequence selected based on the above-mentioned selection of the amino acid residues is that in which Lys is substituted for Asn35 and/or Glu is substituted for Asp36 and in which an amino acid sequence except the positions where the substitution is executed is the same as the amino acid sequence of each cell membrane domain of the wild-type protein G. Consequently, the first aspect of the mutant proteins is distinguished from the following mutant protein with the improved stability of the cell membrane domain of the protein G claimed by the inventors.

Specific examples of the mutant proteins according to the present invention include the following a) to c).
a) A mutant protein of B 1 domain protein of the wild-type protein G consisting of an amino acid sequence represented by (a) or of the amino acid sequence obtained by deleting, substituting, inserting or adding one or several amino acid residues in the amino acid sequence represented by (a), wherein
   the mutant protein has the binding property to the Fc region of immunoglobulin G, and
   the mutant protein has at least the binding property to the Fab region of immunoglobulin G and/or the binding property to the Fc region in the weakly acidic region is decreased in comparison with a B 1 domain protein of the wild-type protein G. 
(a)AspThrTyrLysLeuIleLeuAsnGlyLysX11LeuLysGlyGluThrX17ThrGluAlaValX22Ala
AlaX25AlaGluLysValPheLysX32TyrAlaX35X36X37GlyValX40GlyX42TrpThrTyrAspX47X4
8ThrLysThrPheThrValThrGlu
   (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or Leu; X47 is Asp or Pro; X48 is Ala, Lys or Glu; X22 is Asp; X25 is Thr; X32 is Gln; X40 is Asp; X42 is Glu; and X11 is Thr, and X17 is Thr simultaneously.)
b) A mutant protein of B2 domain protein of the wild-type protein G consisting of an amino acid sequence represented by (b) or of the amino acid sequence obtained by deleting, substituting, inserting or adding one or several amino acid residues in the amino acid sequence represented by (b), wherein
   the mutant protein has the binding property to the Fc region of immunoglobulin G, and
   the mutant protein has at least the binding property to the Fab region of immunoglobulin G and/or the binding property to the Fc region in the weakly acidic region is decreased in comparison with B2 domain protein of the wild-type protein G. (b)ThrThrTyrLysLeuValIleAsnGlyLysX11LeuLysGlyGluThrX17ThrGluAlaValX22Ala 
AlaX25AlaGluLysValPheLysX32TyrAlaX35X36X37GlyValX40GlyX42TrpThrTyrAspX47X4
8ThrLysThrPheThrValThrGlu
 (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; X42 is Glu; and X11 is Thr and X17 is Thr simultaneously.)
c) mutant protein of B3 domain protein of the wild-type protein G consisting of an amino acid sequence represented by (c) or of the amino acid sequence obtained by deleting, substituting, inserting or adding one or several amino acid residues in the amino acid sequence represented by (c), wherein
   the mutant protein has the binding property to the Fc region of immunoglobulin G, and
   the mutant protein has at least the binding property to the Fab region of immunoglobulin G and/or the binding property to the Fc region in the weakly acidic region is decreased in comparison with B3 domain protein of the wild-type protein G. 
(c)ThrThrTyrLysLeuValIleAsnGlyLysX11LeuLysGlyGluThrX17ThrLysAlaValX22Ala
GluX25AlaGluLysAlaPheLysX32TyrAlaX35X36X37GlyValX40GlyValTrpThrTyrAspX47X4
8ThrLysThrPheThrValThrGlu
   (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; and X11 is Thr and X17 is Thr simultaneously.)
   In the above-mentioned definitions of the amino acid residues (a) to (c), the provisos are for distinguishing the amino acid residue from each cell membrane domain protein of the wild-type protein G and the following mutant protein with the improved stability of the cell membrane domain of the protein G claimed by the inventors.
   Further specific examples of the mutant proteins according to the present invention include the following d) to i).
d) A mutant protein of B 1 domain protein of the wild-type protein G consisting of an amino acid sequence represented by (d) or of the amino acid sequence obtained by deleting, substituting, inserting or adding one or several amino acid residues in the amino acid sequence represented by (d), wherein
   the mutant protein has the binding property to the Fc region of immunoglobulin G, and the mutant protein has the binding property to the Fab region of immunoglobulin G and/or the binding property to the Fc region in the weakly acidic region is decreased in comparison with B 1 domain protein of the wild-type protein G. 
(d)AspThrTyrLysLeuIleLeuAsnGlyLysX11LeuLysGlyGluThrX17ThrGluAlaValX22AlaAla
X25AlaGluLysValPheLysX32TyrAlaAsnAspAsnGlyValX40GlyX42TrpThrTyrAspAspAlaThrLys
ThrPheThrValThrGlu
   (In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)
e) A mutant protein of B2 domain protein of the wild-type protein G consisting of an amino acid sequence represented by (e) or of the amino acid sequence obtained by deleting, substituting, inserting or adding one or several amino acid residues in the amino acid sequence represented by (e), wherein
   the mutant protein has the binding property to the Fc region of immunoglobulin G, and
   the mutant protein has the binding property to the Fab region of immunoglobulin G and/or the binding property to the Fc region in the weakly acidic region is decreased in comparison with B2 domain protein of the wild-type protein G. 
(e)ThrThrTyrLysLeuValIleAsnGlyLysX11LeuLysGlyGluThrX17ThrGluAlaValX22AlaAla
X25AlaGluLysValPheLysX32TyrAlaAsnAspAsnGlyValX40GlyX42TrpThrTyrAspAspAlaThrLys
ThrPheThrValThrGlu
   (In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)
f) A mutant protein of B3 domain protein of the wild-type protein G consisting of an amino acid sequence represented by (f) or of the amino acid sequence obtained by deleting, substituting, inserting or adding one or several amino acid residues in the amino acid sequence represented by (f), wherein
   the mutant protein has the binding property to the Fc region of immunoglobulin G, and
   the mutant protein has the binding property to the Fab region of immunoglobulin G and/or the binding property to the Fc region in the weakly acidic region is decreased in comparison with B3 domain protein of the wild-type protein G. 
(f)ThrThrTyrLysLeuValIleAsnGlyLysX11LeuLysGlyGluThrX17ThrLysAlaValX22Ala
GluX25AlaGluLysAlaPheLysX32TyrAlaAsnAspAsnGlyValX40GlyValTrpThrTyrAspAspAla
ThrLysThrPheThrValThrGlu
   (In the amino acid sequence, X22 represents Asp or His; X25 represents Thr or His; X32 represents Gln or His; X40 represents Asp or His; X11 represents Thr or Arg; and X17 represents Thr or Ile, respectively, with the proviso that a case is excluded where X22 is Asp; X25 is Thr; X32 is Gln; X40 is Asp; and X11 is Thr and X17 is Thr simultaneously.)
g) A mutant protein of B 1 domain protein of the wild-type protein G consisting of an amino acid sequence represented by (g) or of the amino acid sequence obtained by deleting, substituting, inserting or adding one or several amino acid residues in the amino acid sequence represented by (g), wherein
   the mutant protein has the binding property to the Fc region of immunoglobulin G, and
   the mutant protein has the binding property to the Fc region in the weakly acidic region is decreased in comparison with B 1 domain protein of the wild-type protein G. 
(g)AspThrTyrLysLeuIleLeuAsnGlyLysThrLeuLysGlyGluThrThrThrGluAlaValX22Ala
AlaX25AlaGluLysValPheLysX32TyrAlaAsnAspAsnGlyValX40GlyX42TrpThrTyrAspAspAla
ThrLysThrPheThrValThrGlu
   (In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)
h) Each of mutant proteins of B2 domain protein of the wild-type protein G consisting of an amino acid sequence represented by (h) or of the amino acid sequence obtained by deleting, substituting, inserting or adding one or several amino acid residues in the amino acid sequence represented by (h), wherein
   the mutant protein has the binding property to the Fc region of immunoglobulin G, and
   the mutant protein has the binding property to the Fc region in the weakly acidic region is decreased in comparison with B2 domain protein of the wild-type protein G. 
(h)ThrThrTyrLysLeuValIleAsnGlyLysThrLeuLysGlyGluThrThrThrGluAlaValX22AlaA
laX25AlaGluLysValPheLysX32TyrAlaAsnAspAsnGlyValX40GlyX42TrpThrTyrAspAspAlaT
hrLysThrPheThrValThrGlu
   (In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)
i) Each of mutant proteins of B3 domain protein of the wild-type protein G consisting of an amino acid sequence represented by (i) or of the amino acid sequence obtained by deleting, substituting, inserting or adding one or several amino acid residues in the amino acid sequence represented by (i), wherein
   the mutant protein has the binding property to the Fc region of immunoglobulin G, and
   the mutant protein has the binding property to the Fc region in the weakly acidic region is decreased in comparison with B3 domain protein of the wild-type protein G. 
(i)ThrThrTyrLysLeuValIleAsnGlyLysThrLeuLysGlyGluThrThrThrLysAlaValX22AlaGl
uX25AlaGluLysAlaPheLysX32TyrAlaAsnAspAsnGlyValX40GlyValTrpThrTyrAspAspAlaThr
LysThrPheThrValThrGlu
   (In the amino acid sequence, X22 represents Asp or His; X25 represents Thr or His; X32 represents Gln or His; and X40 represents Asp or His, respectively, with the proviso that a case is excluded where X22 is Asp; and X25 is Thr; X32 is Gln and X40 is Asp simultaneously.)

In the above-mentioned definitions of the amino acid residues (d) to (i), the provisos are for distinguishing the amino acid residue from each cell membrane domain protein of the wild-type protein G.

As is clear from above, in the design of the mutant proteins of the present invention, the target part for the mutation which is selected and the amino acid residue which is substituted for the part are not limited to only one of each, so that the amino acid sequence of the mutant protein can be designed by appropriately selecting from the target parts for the mutation and the amino acid residues which are substituted for the parts. For example, to improve the binding property to the Fc region of immunoglobulin G, a plurality of amino acid sequences of the mutant proteins can be designed by the steps of; selecting Asp22, Thr25, Gln32, Asp40 and Glu42 in the amino acid sequence of the B 1 or B2 domain of the wild-type protein G as the target parts for the mutation, selecting Asp22His, Thr25His, Gln32His, Asp40His and Glu42His as the corresponding amino acid residues which are substituted, and executing point mutation or multiplex mutation of up to five mutation positions / five substitutions based on any one of amino acid substitutions or a combination of the amino acid substitutions to the wild-type amino acid sequence of the B 1 or B2 domain of the protein G (SEQ ID NO. 1, 2). The above-mentioned amino acid sequences in (g) and (h) represent such point mutation and such multiplex mutation of up to five mutation positions / five substitutions, and are an example of the mutant proteins of the present invention.

Also, for example, the mutant protein in which the improvement of the binding property to the Fab region of immunoglobulin G is further applied in addition to the above-mentioned improvement of the binding property to the Fc region of immunoglobulin G includes mutant proteins designed by the steps of; selecting Thr11 and Thr17 in the B 1 or B2 domain of the wild-type protein G, selecting Thr11Arg and Thr17Ile as the corresponding amino acid residues, and executing mutation of up to seven mutation positions / seven substitutions, in which these two options are added and transduced to the above-mentioned mutation of up to five mutation positions / five substitutions, to the wild-type amino acid sequence of the B1 or B2 domain of the protein G. The above-mentioned amino acid sequences in (d) and (e) represent examples of such point mutation or such multiplex mutation of up to seven mutation positions / seven substitutions, and, in the amino acid sequences, amino acid sequence with mutation of Thr11Arg and/or Thr17Ile and with any one or more of the above-mentioned mutation of Asp22His, Thr25His, Gln32His, Asp40His and Glu42His is that in which the improvement of the binding property to the Fab region of immunoglobulin G is further applied in addition to the improvement of the binding property to the Fc region of immunoglobulin G.

On the other hand, although the above-mentioned amino acid sequence in (i) is an example of an amino acid sequence of a B3 domain mutant protein of the wild-type protein G, it is designed similarly as the amino acid sequences in (g) and (h), except for mutation of up to four mutation positions / four substitutions of any one or more of Asp22His, Thr25His, Gln32His and Asp40His as the above-mentioned mutation for improving the binding property to the Fc region. Also, although the above-mentioned amino acid sequence in (f) is an example of the amino acid sequence of the B3 domain mutant protein of the wild-type protein G, it is designed similarly as the amino acid sequences in (d) and (e), except for mutation of up to six mutation positions / six substitutions of any one or more of Asp22His, Thr25His, Gln32His and Asp40His, as the above-mentioned mutation for improving the binding property to the Fc region, and Thr11Arg and Thr17Ile, as the above-mentioned mutation for improving the binding property to the Fab region.

In the present invention, the mutation which has been already known to make the property of the extracellular domain of the protein G more preferable may be further applied in addition to such mutation. For example, a mutation method for improving the thermal stability, the chemical resistance to a denaturing agent and the resistance to a decomposing enzyme of the extracellular domain of the protein G has been found through previous research by the inventors (Patent Document 6). Namely, transduction of mutation of any one or more of Asn35Lys, Asp36Glu, Asn37His, Asn37Leu, Asp47Pro, Ala48Lys and Ala48Glu improves the above-mentioned stability of the B1, B2 or B3 domain of the protein G. By combining this mutation with the above-mentioned mutation for improving a binding characteristic to the Fc region of immunoglobulin G and/or a binding characteristic to the Fab region thereof according to the present invention, the mutant proteins of the present invention become more useful.

For example, more stabilized amino acid sequence of a plurality of mutant proteins can be designed by the step of executing multiplex mutation of up to twelve mutation positions / fourteen substitutions, in which this mutation for the stabilization is added and transduced to the above-mentioned mutation of up to seven mutation positions / seven substitutions, to the wild-type amino acid sequence of the B 1 or B2 domain of the protein G (SEQ ID NO. 1, 2).

Although the above-mentioned amino acid sequences in (a) and (b) represent such point mutation and such multiplex mutation of up to twelve mutation positions / fourteen substitutions, the wild-type sequence as well as the mutation only for the stabilization which is applied to the wild-type sequence are excluded.

Transduction of mutation of any one or more of Asn35Lys, Asp36Glu, Asn37His, Asn37Leu, Asp47Pro, Ala48Lys and Ala48Glu in the amino acid sequences in (a) and (b) improves the stability of the B1 or B2 domain mutant protein of the protein G in addition to the above-mentioned improvement of the binding characteristic to the Fab region of immunoglobulin G and/or the binding characteristic to the Fc region thereof in the weakly acidic region as the effect of the mutation of up to seven mutation positions / seven substitutions.

On the other hand, although the above (c) describes an example of the amino acid sequence of the B3 domain mutant protein of the wild-type protein G and point mutation and multiplex mutation of up to eleven mutation positions / thirteen substitutions, it is designed similarly as the amino acid sequences in (a) and (b), except for mutation of up to four mutation positions / four substitutions of any one or more of Asp22His, Thr25His, Gln32His and Asp40His as the above-mentioned mutation for improving the binding property to the Fc region. Therefore, transduction of mutation of any one or more of Asn35Lys, Asp36Glu, Asn37His, Asn37Leu, Asp47Pro, Ala48Lys and Ala48Glu in the amino acid sequence in (c) also improves the stability of the B3 domain mutant protein of the protein G in addition to the improvement of the binding characteristic to the Fab region of immunoglobulin G and/or the binding characteristic to the Fc region thereof in the weakly acidic region.

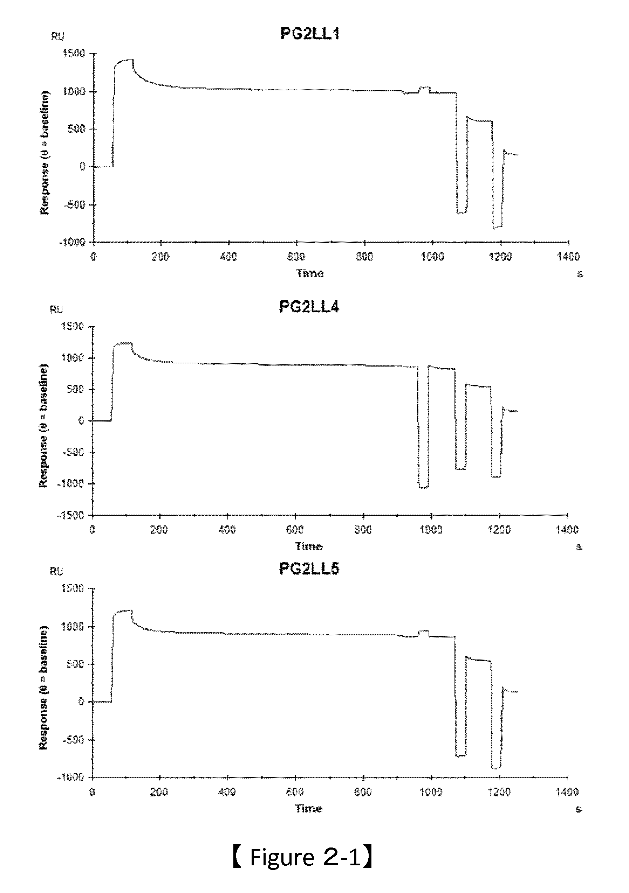

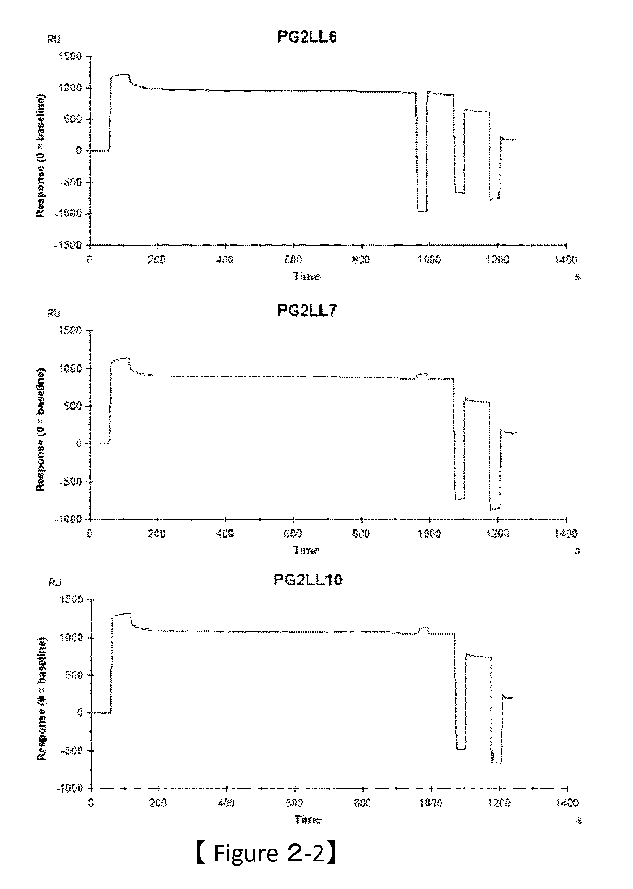

As described above, the target parts for the mutation in the present invention are selected by using each three-dimensional atomic coordinate data of the B2 domain of the protein G - Fc complex and the B3 domain thereof - Fab complex, but since the B 1 domain hardly differs from the B2 domain in not only the amino acid sequences (FIG. 2) but also the stereoscopic structure, the above-mentioned selected mutation is effective equally for each domain. Also, since the B3 domain hardly differs from the B2 domain in not only the amino acid sequences (FIG. 2) but also the stereoscopic structure, the above-mentioned mutation selected in the B2 domain is effective equally for each domain.

For example, all the above-mentioned wild-type amino acid residues in the selected twelve mutation positions are common between the above-mentioned B2 domain and the above-mentioned B 1 domain. Therefore, the point mutation and the multiplex mutation based on the combination of the above-mentioned selected five mutation positions / five substitutions, seven mutation positions / seven substitutions or twelve mutation positions / fourteen substitutions can be transduced to the B1 amino acid sequence which is highly equal to the B2 domain to transduce the amino acid sequence of the mutant protein of the B1 domain. Also, all the above-mentioned wild-type amino acids in the selected twelve mutation positions except the 42nd position are common between the above-mentioned B2 domain and the above-mentioned B3 domain (the wild-type amino acid residue in the 42nd position is Glu42 in the B2 domain and is Val42 in the B3 domain). Therefore, the point mutation and the multiplex mutation based on the combination of the four mutation positions / four substitutions, six mutation positions / six substitutions or eleven mutation positions / thirteen substitutions, in which only the mutation position in the 42nd position is excluded from the above-mentioned selected five mutation positions / five substitutions, seven mutation positions / seven substitutions or twelve mutation positions / fourteen substitutions, can be transduced to the amino acid sequence of the B3 domain which is highly equal to the B2 domain to produce the amino acid sequence of the mutant protein of the B3 domain. This is also clear from the fact that, as shown in the following examples, the mutant proteins of the B1 domain based on the selection by using each three-dimensional atomic coordinate data of the B2 domain of the protein G - Fc complex and the B3 domain thereof - Fab complex have performance as intended.

As described above, the amino acid sequence of the mutant proteins of the present invention is not limited to one, and there exist a plurality of amino acid sequences among which preferable sequences specifically include an amino acid sequence represented by [SEQ ID NO. 13], [SEQ ID NO. 14], [SEQ ID NO. 15], [SEQ ID NO. 16], [SEQ ID NO. 17], [SEQ ID NO. 18], [SEQ ID NO. 19] or [SEQ ID NO. 20].

As for the mutant protein represented by [SEQ ID NO. 13], mutation is transduced to the part, with respect to which, through the previous research, the inventors have found that the thermal stability, the chemical resistance to a denaturing agent and the resistance to a decomposing enzyme of the extracellular domain of the protein G can be improved, in the wild-type amino acid sequence of the B1 domain of the protein G represented by [SEQ ID NO. 1], and, as for the mutant proteins represented by [SEQ ID NO. 14], [SEQ ID NO. 15], [SEQ ID NO. 19] and [SEQ ID NO. 20], mutation is further transduced to the part which is selected based on the analysis of the surface bound to the Fc.

On the other hand, as for the mutant proteins represented by [SEQ ID NO. 16], [SEQ ID NO. 17] and [SEQ ID NO. 18], mutation is transduced to the part, with respect to which, through the previous research, the inventors have found that the thermal stability, the chemical resistance to a denaturing agent and the resistance to a decomposing enzyme of the extracellular domain of the protein G can be improved, and to the part which is selected based on the analysis of the surface bound to the Fab, in the wild-type amino acid sequence of the B 1 domain of the protein G represented by [SEQ ID NO. 1].

Although the mutant proteins of the present invention have the binding property to the protein having the antibody, the immunoglobulin G or the Fc region of immunoglobulin G, mutation such as deletion, substitution, insertion, or addition may be generated in relation to one or several (for example, two to five) amino acid residues of the amino acid sequence described above as any one of the mutant proteins of the present invention as long as at least the binding property to the Fab region of immunoglobulin G and/or the binding property to the Fc region thereof in the weakly acidic region is decreased in comparison with each extracellular domain protein of the wild-type protein G, so that the sequence identity of the mutant proteins to each amino acid sequence as a reference is more than 90 %, preferably more than 95 %, and more preferably more than 98 %.

An example of the domain mutant comprised in the protein used in the following examples is a polypeptideconsisting of the amino acid sequences represented by SEQ ID NO. 19, as described in the following examples:

(j)AspThrTyrLysLeuIleLeuAsnGlyLysThrLeuLysGlyGluThrThrThrGluAlaValAspAlaAlaThrAlaGl
uLysValPheLysHisTyrAlaAsnGluHisGlyValHisGlyHisTrpThrTyrAspProGluThrLysThrPheThrValT
hrGlu.

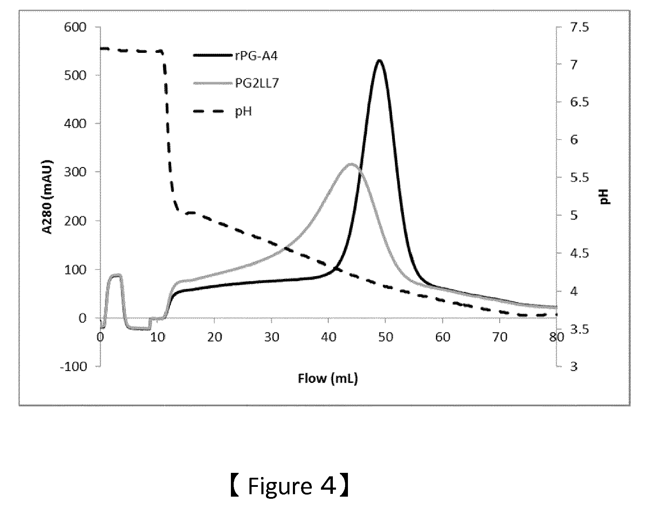

Also, the protein of the present invention may be a fusion protein consisting of a fusion-type amino acid sequence, in which an amino acid sequence of any other proteins is connected to an n-terminal side or a c-terminal side. For example, the protein may be [an amino acid sequence (a)] - a linker sequences E - a protein A, or a protein B - a linker sequences F - [an amino acid sequence (a)] - a linker sequences G - a protein C - a linker sequences H - [an amino acid sequence (c)]. The other amino acid sequences used in such a fusion protein include, for example, an amino acid sequence of an oxaloacetate decarboxylase alpha-subunit c-terminal domain (OXADac) shown in FIG. 4 or represented by [SEQ ID NO. 31]. As shown in the following examples, an OXADac - protein G mutant fusion protein in this case can have a plurality of functions of avidin binding property resulting from the OXADac region and the antibody binding property resulting from the protein G mutant region in a single molecule.

For example, in the case of synthesis of the protein of the present invention in a form of a fusion protein with the His tag or other proteins, even if the fusion protein is decomposed between the tag and a mutant protein or between the other proteins and the protein of the present invention with a sequence-specific proteolytic enzyme after the synthesis, one or several amino acid residues may remain in the n-terminal side or the c-terminal side of the protein of the present invention, and also, in the case of production of the protein of the present invention with an Escherichia coli or the like, a methionine or the like corresponding to an initiation codon may be added to the n-terminal side, but the addition of these amino acid residues does not change the following activity of the protein of the present invention. Moreover, the addition of these amino acid residues does not neutralize effects of designed mutation. Therefore, the protein of the present invention contains the mutation naturally. In order to prepare the protein of the present invention without the addition of such amino acid residues, the protein is produced, for example, with the Escherichia coli or the like, and furthermore the amino acid residue of the N-terminal is cut selectively with an enzyme such as methionyl aminopeptidase or the like (Reference Document 7) to separate and purify the protein from a reaction mixture by the chromatography or the like.

The "protein "in the present specification means not only the protein constituted by linking with linkers the plural domains having affinity for the protein comprising Fc region of the immunoglobulin G (IgG) but also the above fusion protein. The present invention also relates to the nucleic acid encoding the protein, the recombinant vector comprising the vector, and the transformant into which the recombinant vector is introduced (transformed).

The present invention further relates to the capturing agent for the immunoglobulin G, or the protein comprising the Fc or Fab region of the immunoglobulin G (referred to hereinafter as "immunoglobulin G and the like"), wherein the protein according to the present invention is immobilized to a water-insoluble solid support; to the affinity chromatography comprising said capturing agent for the purification of antibody, immunoglobulin G, or the protein comprising the Fc or Fab region of the immunoglobulin G, and to the method for the purification of immunoglobulin G, or the protein comprising the Fc or Fab region of the immunoglobulin G by means of said affinity chromatography. The term "having affinity for" the immunoglobulin G and the like means their capable of absorbing the immunoglobulin G and the like in the chromatography. As shown by the following examples, as said capturing agent can sufficiently absorb some kinds of the immunoglobulin G such as the Fab fragment of human IgG and rat IgG, it is very useful as a capturing agent for the purification for them.

As the protein according to the present invention is used as the capturing agent for the antibody in the present method for the purification, it is preferable to use an affinity chromatography of a column such as glass tube that is filled with said capturing agent wherein said protein is immobilized to a water-insoluble carrier (a water-insoluble solid support) represented by, for example, agarose beads.

A buffer of around a neutral pH is used as an absorbing buffer, and any kinds of salts may be used as long as its pH can be adjusted, being phosphate buffer and tris buffer comprising electrolytes such as sodium chloride dissolved therein. The pH of the absorbing buffer may be pH 9.0-6.5, preferably pH 8.0-7.0. The pH range of an eluting buffer will be those under which the desired immunoglobulin G and the like can elute, being pH 6.5-2.0. Any kinds of salts known for those skilled in the art may be used for the eluting buffer as long as its pH can be adjusted, being phosphate, citrate, acetate, and glycine buffers.

The operations per se in the method according to the present invention can be performed in usual manners well known in the art. Thus, like in the usual affinity purification, first a sample solution comprising immunoglobulin G and the like to be purified is injected into a column stabilized with an absorbing buffer so that the immunoglobulin G and the like are absorbed to the fillers. Then, after non-absorbed components remaining in the column are washed out with the absorbing buffer, the absorbed immunoglobulin G and the like is eluted with an eluting buffer to collect the immunoglobulin G and the like in the solution. Those skilled in the art may optionally determine conditions of the affinity purification such as a flow rate of the absorbing and eluting buffers and temperature of the column.

There is no limitation on the origin and components of the sample solution as long as it comprises the immunoglobulin G and the like, which may be serum and culturing liquid of ascites. Any means known for those skilled in the art may be used for the purification in the present method, such as immuno-precipitation method and magnetic beads having the protein of the present invention absorbed thereto, as long as it utilizes the affinity between the protein comprising the domain mutant (artificially mutated domain) and the immunoglobulin G and the like.

The mutant protein described in patent Document 8 is listed as a preferable example of the mutant of the B 1 domain of the wild-type protein G, which is comprised in the protein according to the present invention. Such mutant protein may be easily prepared by those skilled in the art in accordance with the methods described in Patent Document 7 or Patent Document 8, for example, by the following methods.

### Production of the protein

### (1) Production of the protein by a genetic engineering technique

### a. Gene encoding the mutant protein

In the present invention, a genetic engineering method can be used to produce the above-mentioned designed protein.

A gene used in such a method consists of a nucleic acid encoding the protein described in the above A to C, more specifically, encoding the amino acid sequence described in any one of the above (a) to (i), or consists of a nucleic acid encoding a protein which has an amino acid sequence obtained by deleting, substituting or adding one or several amino acid residues in the amino acid sequence described in any one of (a) to (i) and which has the binding property to the protein having the antibody, the immunoglobulin G or the Fc region of immunoglobulin G, wherein the binding property is decreased in the weakly acidic region in comparison with in the neutral region; and, more specifically, the nucleic acid consists of a base sequence represented by any one of [SEQ ID NO. 22] to [SEQ ID NO. 29], for example.

Moreover, a gene used in the present invention also includes a nucleic acid hybridizing with a nucleic acid which consists of a sequence complementary to the above-mentioned base sequence of the nucleic acid under a stringent condition, and encoding the above-mentioned mutant protein which has the binding property to the protein having the antibody, the immunoglobulin G or the Fc region of immunoglobulin G, wherein the binding property to the Fab region of immunoglobulin G and/or the binding property to the Fc region thereof in the weakly acidic region is decreased in comparison with each corresponding extracellular domain protein of the wild-type protein G. The stringent condition herein refers to a condition that a specific hybrid is formed and that a non-specific hybrid is not formed. Specifically, it refers to a condition that a nucleic acid with high identity (the identity is more than 60 %, preferably more than 80 %, more preferably more than 90 %, and most preferably more than 90 %) hybridizes. More specifically, it refers to a condition that sodium concentration is 150 mM to 900 mM, and preferably 600 mM to 900 mM and that temperature is 60 °C to 68 °C, and preferably 65 °C. If hybridization by a conventional means, such as Southern blot, dot blot hybridization, is confirmed, for example, under a hybridization condition of 65 °C and a washing condition of 65 °C, for ten minutes, in 0.1xSSC containing 0.1 % SDS, it can be called "hybridizing under the stringent condition".

The gene encoding the protein of the present invention includes a nucleic acid encoding the above-mentioned nucleic acid and the above-mentioned optional linker sequences, depending on the desired structure of the protein of the present invention. A plurality of nucleic acids which encode each mutant protein constituting the tandem-type multimer and a plurality of nucleic acids encoding the linker sequences may be alternately connected, or the nucleic acid may be designed to encode a fusion-type amino acid sequence by connecting the above-mentioned nucleic acid and a nucleic acid encoding an amino acid sequences of any protein.

### b. Gene, recombinant vector and transformant

The above-mentioned gene of the present invention can be synthesized by a chemical synthesis, a PCR, a cassette mutagenesis, a site-specific mutagenesis or the like. For example, a plurality of oligonucleotides up to about 100 bases with a complementary region of about 20 base pair at the terminal are chemically synthesized, and then by combining the oligonucleotides to perform the overlap extension method (Reference Document 8), the desired gene can be totally synthesized.

The recombinant vector of the present invention can be obtained by connecting (inserting) the gene comprising the above-mentioned base sequence to an appropriate vector. The vector used herein is not particularly limited as long as it is replicable in a host or it can incorporate the desired gene into a host genome. For example, the vector includes a bacteriophage, a plasmid, a cosmid, a phagemid and the like.

A plasmid DNA includes a plasmid derived from actinomycetes (such as pK4, pRK401 and pRF31), a palasmid derived from the Escherichia coli (such as pBR322, pBR325, pUC118, pUC119 and pUC18), a plasmid derived from hay bacillus (such as pUB110 and pTP5), a plasmid derived from yeast (such as YEp13, YEp24 and YCp50), and the like; and a phage DNA includes a λ phage (such as λgt10, λgt11 and λZAP). Moreover, an animal virus vector such as a retrovirus or a vaccinia virus and an insect virus vector such as a baculovirus may be used.

For inserting the gene into the vector, a method in which first a purified DNA is cut with an appropriate restriction enzyme and next the gene is inserted into a restriction enzyme site or a multicloning site of an appropriate vector DNA and connected with the vector, or the like is adopted. The gene must be incorporated into the vector so that the mutant protein of the present invention is expressed. Therefore, in addition to a promoter and the base sequence of the gene, a cis element such as an enhancer, a splicing signal, a poly A addition signal, a selection marker, a ribosome-binding sequence (an SD sequence), an initiation codon, a termination codon, and the like may be optionally connected to the vector of the present invention. Also, a tag sequence for facilitating purification of the protein which is produced may be connected, As the tag sequence, a base sequence encoding the known tag such as His tag, GST tag, MBP tag and BioEase tag may be used.

A confirmation as to whether the gene is inserted into the vector can be performed by using the known genetic engineering technology. For example, in the case of the plasmid vector and the like, the confirmation is performed by subcloning the vector with a competent cell to extract DNA and then specifying a base sequence of the DNA with a DNA sequencer. A similar means is available to other vectors as long as they can be subcloned with a bacteria or another host. Also, screening of the vector with the selection marker such as a drug resistant gene is effective.

The transformant can be obtained by introducing the recombinant vector of the present invention to a host cell so that the mutant protein of the present invention can be expressed. The host used for transformation is not particularly limited as long as it can express a protein or a polypeptide. For example, the host includes a bacteria (such as the Escherichia coli and the hay bacillus), a yeast, a plant cell, an animal cell (such as a COS cell and a CHO cell), and an insect cell.

When the bacteria is the host, it is preferable that the recombinant vector is autonomously replicable in the bacteria and, in addition, that the bacteria is constituted by the promoter, the ribosome-binding sequence, the initiation codon, the nucleic acid encoding the mutant protein of the present invention and a transcription termination sequence. For example, the Escherichia coli includes an Escherichia coli BL21 and the like, and the hay bacillus includes a Bacillus subtilis and the like. A method for transducing the recombinant vector to the bacteria is not particularly limited as long as it is a method for transducing DNA to bacteria.

For example, the method includes a heat shock method, a method using a calcium ion, an electroporation method and the like.

When the yeast is the host, for example, a Saccharomyces cerevisiae, a Schizosaccharomyces pombe or the like is used. A method for transducing the recombinant vector to the yeast is not particularly limited as long as it is a method for transducing DNA to a yeast, and, for example, the method includes the electroporation method, a spheroplast method, a lithium acetate method and the like.

When the animal cell is the host, a monkey cell COS-7, a Vero cell, a chinese hamster ovarian cell (a CHO cell), a mouse L cell, a rat GH3, a human FL cell or the like is used. For example, a method for transducing the recombinant vector to the animal cell includes the electroporation method, a calcium phosphate method, a lipofection method and the like. When the insect cell is the host, a Sf9 cell or the like is used. For example, a method for transducing the recombinant vector to the insect cell includes the calcium phosphate method, the lipofection method, the electroporation method and the like.

A confirmation as to whether the gene is transduced to the host can be performed by using a PCR method, a southern hybridization method, a northern hybridization method or the like. For example, DNA is prepared from the transformant, and then a DNA-specific primer is designed to perform the PCR. Next, an amplification product of the PCR is subjected to an agarose gel electrophoresis, a polyacrylamide gel electrophoresis, a capillary electrophoresis or the like and is stained with an ethidium bromide, a SyberGreen solution or the like; and then, through detecting the amplification product as one band, it can be confirmed that the transformation has been performed. Alternatively, by using a primer previously labeled with a fluorescent pigment or the like, the PCR may also be performed to detect an amplification product.

### c. Acquisition of the protein by culturing the transformant

When the protein of the present invention is produced as a recombinant protein, it can be obtained by culturing the above-mentioned transformant and then collecting the protein from the cultured product. The cultured product refers to any one of a culture supernatant, a cultured cell, or a cultured cell body; and a disrupted product of a cell or a cell body. A method for culturing the transformant of the present invention is performed according to a conventional method used for culture of a host.

A medium for culturing a transformant obtained by using a microorganism, such as the Escherichia coli or a yeast fungus, as the host may be any one of a natural medium and a synthesis medium as long as it contains a carbon source, a nitrogen source, inorganic salts or the like which can be assimilated by the microorganism, and is a medium which can effectively culture a transformant. The carbon source includes a carbohydrate such as glucose, fructose, sucrose and starch; an organic acid such as acetic acid and propionic acid; and alcohols such as ethanol and propanol. The nitrogen source includes not only ammonia; an ammonium salt of an inorganic acid or an organic acid such as ammonium chloride, ammonium sulfate, ammonium acetate and ammonium phosphate; or other nitrogen-containing compounds; but also peptone, meat extract, corn steep liquor and the like. An inorganic substance includes monopotassium phosphate, dipotassium phosphate, magnesium phosphate, magnesium sulfate, sodium chloride, ferrous sulfate, manganese sulfate, copper sulfate, calcium carbonate and the like. The culture is normally performed under an aerobic condition of shake culture, aeration-agitation culture or the like, at 20 °C to 37 °C, for 12 hours to for 3 days.

After the culture, when the protein of the present invention is produced in the cell body or in the cell, the cell body or the cell is crushed by performing ultrasonic treatment, repetition of freezing and thawing, homogenizer treatment or the like to collect the protein. Alternatively, when the protein is produced out of the cell body or out of the cell, a culture solution is used as it is, or the cell body or the cell is removed by centrifugal separation or the like. Then, by using a general biochemical method for isolation and purification of a protein, such as ammonium sulfate precipitation, gel chromatography, ion exchange chromatography, affinity chromatography, alone or in proper combination, the protein of the present invention can be isolated and purified from the above-mentioned cultured product.

Moreover, by utilizing a so-called cell-free synthesis system in which only factors concerning biosynthesis reaction of a protein (such as an enzyme, the nucleic acid, an ATP, the amino acid) are mixed, the mutant protein of the present invention can be synthesized from the vector without using a living cell, in vitro (Reference Document 9). Then, by using a purification method similar to the above, the mutant protein of the present invention can be isolated and purified from a mixed solution after the reaction.

To confirm whether the protein of the present invention obtained by the isolation and purification is a protein consisting of the desired amino acid sequence, a sample containing the protein is analyzed. As an analysis method, the SDS-PAGE, a western blotting, a mass spectrometry, amino acid analysis, an amino acid sequencer and the like can be used (Reference Document 10).

### (2) Production of the protein by other means

The protein of the present invention may be produced by an organic chemical means such as a solid phase peptide synthesis method. The production method of the protein using such a means is well known in this technical field, and thus is concisely described below.

When the protein is chemically produced by the solid phase peptide synthesis method, protecting polypeptide with the amino acid sequence of the protein of the present invention is synthesized on a resin by repeating polycondensation reaction of an activated amino acid derivative, preferably with an automatic synthesizer. Next, at the same time that the protecting polypeptide is cleaved from the resin, the protecting groups of side-chains are also cleaved. It is known that, for the cleavage reaction, there exists a suitable cocktail depending on kinds of the resin and the protecting groups, and composition of the amino acids (Reference Document 11). Then, a roughly purified protein is transferred from an organic solvent layer to an aqueous layer, and the target protein is purified. As the purification method, reversed-phase chromatography or the like can be used (Reference Document 11).

### 2. Immobilization of the protein

The protein used in the method for the purification according to the present invention is used as the capturing agent for the antibodies and the like by utilizing its antibody binding property.

While the capturing agent of the present invention may be of any formation as long as it comprises the protein according to the present invention, it is preferably formulated so that the mutant protein according to the present invention is immobilized to the water-insoluble solid support. A water-insoluble carrier used herein includes an inorganic carrier such as a glass bead and silica gel; an organic carrier consisting of a synthesis polymer such as cross-linked polyvinyl alcohol, cross-linked polyacrylate, cross-linked polyacrylamide and cross-linked polystyrene, or polysaccharide such as crystalline cellulose, cross-linked cellulose, cross-linked agarose and cross-linked dextran; a composite carrier such as organic-organic and organic-inorganic obtained by combinations thereof; or the like; among which a hydrophilic carrier is preferable since the nonspecific absorption is relatively little and the selectivity to the antibody, the immunoglobulin G or the protein having the Fc region of immunoglobulin G is excellent. The hydrophilic carrier as used herein refers to a carrier in which a contact angle with water is 60° or less in the case that the compound constituting the carrier is formed into a flat plate. A typical example of such a carrier includes a carrier consisting of polysaccharide such as cellulose, chitosan and dextran, polyvinyl alcohol, saponified ethylene - vinyl acetate copolymer, polyacrylamide, polyacrylic acid, polymethacrylic acid, polymethyl methacrylate, polyacrylic acid-grafted polyethylene, polyacrylamide-grafted polyethylene, glass, or the like.

Commercial products include GCL2000 and GC700 which are porous cellulose gel, Sephacryl S-1000 in which allyl dextran and methylenebisacrylamide are cross-linked by covalent bonds, Toyopearl which is an acrylate-based carrier, SepharoseCL4B which is an agarose-based cross-linked carrier, Eupergit C250L which is polymethacrylamide activated with epoxy groups, and the like. However, the carrier in the present invention is not limited to only these carriers and activated carriers. The above-mentioned carriers may be each independently used, or may be used as a mixture of any two or more thereof. Moreover, based on the purposes and methods for using the antibody capturing agent, the water-insoluble carrier used herein has desirably a wide surface area, and has preferably pores of a suitable size, i.e. porous carrier.

The carrier may be in any form, such as bead-shaped, fiber-shaped and membrane-shaped (including hollow fiber), which can be arbitrarily selected. Due to ease of preparation of a carrier with specific exclusion limit molecular weight, the bead-shaped carrier is particularly preferably used. A bead-shaped carrier with an average particle diameter of 10 µm to 2500 µm is easy to use, and, in particular, from a viewpoint of ease of ligand immobilization reaction, a range from 25µm to 800µm is preferable.

Moreover, it is convenient for the immobilization of ligand that functional groups which can be used for the immobilization reaction of ligand exist on the carrier surface. A typical example of the functional groups includes a hydroxyl group, an amino group, an aldehyde group, a carboxyl group, a thiol group, a silanol group, an amide group, the epoxy group, a succinyl imide group, an acid anhydride groups, an iodoacetyl group, and the like.

In the immobilization of the mutant protein to the above-mentioned carrier, it is more preferable that capture efficiency is improved by decreasing steric hindrance of the mutant protein and further that the mutant protein is immobilized via a hydrophilic spacer to suppress non-specific binding. As the hydrophilic spacer, a derivative of polyalkylene oxide in which, for example, the carboxyl group, the amino group, the aldehyde group, the epoxy group or the like was substituted at the both terminals is preferably used.

Although a method and a condition for immobilizing the mutant protein which is introduced to the above-mentioned solid support (carrier), and organic compounds used as the spacer are not particularly limited, methods generally adopted in the case where the protein and peptide may be listed as its examples.

For example, a method comprising the steps of: reacting the carrier with cyanogen bromide (CNBr), N,N'-disuccinimidyl carbonate (DSC), epoxide and activated carbonic acid (NHS ester) or the like to activate the carrier; (substituting a functional group with which a compound to be immobilized as the ligand is easier to react in comparison with a functional group which the carrier originally has), and reacting the carrier with the compound to be immobilized as the ligand to immobilize; or an immobilization method comprising the step of: adding a condensing reagent, such as carbodiimide, or a reagent which has a plurality of functional groups in a molecule, such as glutaraldehyde, to a system where the carrier and the compound to be immobilized as the ligand exist to condense and crosslink may be included. However, it is more preferable that an immobilization method in which proteins are not detached easily from the carrier during sterilization or use of the capturing agent is applied.

### 3. Performance confirmation test for the protein and the antibody capturing agent

Although the following performance confirmation tests may be performed for the mutant proteins and the proteins produced as described above (hereinafter, also referred to as "the protein" in brief) and the antibody capturing agents to select excellent products, the proteins and the antibody capturing agents of the present invention had all excellent performance.

### (1) Antibody-binding property test

The antibody-binding property of the proteins of the present invention may be confirmed and evaluated by using the western blotting, an immunoprecipitation, a pull-down assay, an ELISA (Enzyme-Linked ImmunoSorbent Assay), the surface plasmon resonance (SPR) method, and the like. Above all, in the SPR method, since interaction between living bodies can be observed over time in real time without label, a binding reaction of the mutant proteins can be evaluated quantitatively from a kinetic viewpoint. Moreover, the antibody-binding property of the mutant protein immobilized to the water-insoluble solid support can be confirmed and evaluated by the above-mentioned SPR method and a liquid chromatography method. Especially, by the liquid chromatography method, the pH dependence relative to the antibody-binding property can be precisely evaluated.

### (2) Thermal stability test for the protein

The thermal stability of the mutant proteins of the present invention may be evaluated by using a circular dichroism (CD) spectrum, a fluorescence spectrum, an infrared spectroscopy, a differential scanning calorimetry, residual activity after heating, and the like. Above all, since the CD spectrum is a spectroscopic analysis method sensitively reflecting change of secondary structure of a protein, a change of the stereoscopic structure of the mutant protein due to temperature can be observed and structural stability can be evaluated quantitatively from a thermodynamic viewpoint.

### [References]

Reference 1: Bjorck L, Kronvall G. (1984) Purification and some properties of streptococcal protein G, a novel IgG-binding reagent. J Immunol. 133, 69-74.
Reference 2: Boyle M. D.P., Ed. (1990) Bacterial Immunoglobulin Binding Proteins. Academic Press, Inc., San Diego, CA.
Reference 3: Gallagher T, Alexander P, Bryan P, Gilliland GL. (1994) Two crystal structures of the B 1 immunoglobulin-binding domain of streptococcal protein G and comparison with NMR. Biochemistry 19, 4721-4729.
Reference 4: Sauer-Eriksson AE, Kleywegt GJ, Uhlen M, Jones TA. (1995) Crystal structure of the C2 fragment of streptococcal protein G in complex with the Fc domain of human IgG. Structure 3, 265-278.
Reference 5: Derrick JP, Wigley DB. (1994) The third IgG-binding domain from streptococcal protein G. An analysis by X-ray crystallography of the structure alone and in a complex with Fab. J Mol Biol. 243, 906-918.
Reference 6: Alexander P, Fahnestock S, Lee T, Orban J, Bryan P. (1992) Thermodynamic analysis of the folding of the streptococcal protein G IgG-binding domains B1 and B2: why small proteins tend to have high denaturation temperatures. Biochemistry 14, 3597-3603.
Reference 7: D'souza VM, Holz RC. (1999) The methionyl aminopeptidase from Escherichia coli can function as an iron (II) enzyme. Biochemistry 38, 11079-11085.
Reference 8: Horton R. M., Hunt H. D., Ho S. N., Pullen J. M. and Pease L. R. (1989). Engineering hybrid genes without the use of restriction enzymes: gene splicing by overlap extension. Gene 77, 61-68.
Reference 9: Masato OKADA, Kaoru MIYAZAKI (2004) Notes for Protein Experiment (first volume). Yodosha Co., Ltd.
Reference 10: Shigeo OHNO, Yoshifumi NISHIMURA (ed.) (1997) Protocol for Protein Experiment 1 - Functional Analysis Part. Shujunsha Co., Ltd.
Reference 11: Shigeo OHNO, Yoshifumi NISHIMURA (ed.) (1997) Protocol for Protein Experiment 2 - Structural Analysis Part. Shunjusha Co., Ltd.

Hereinafter, the present invention will be specifically described with the following examples. However, the technical scope of the present invention is not limited to the following examples. In this specification, various amino acid residues are denoted by the following abbreviations. Ala; an L-alanine residue, Arg; an L-arginine residue, Asp; an L-aspartic acid residue, Asn; an L-asparagine residue, Cys; an L-cysteine residue, Gln; an L-glutamine residue, Glu; an L-glutamic acid residue, Gly; an L-glycine residue, His; an L-histidine residue, Ile; an L-isoleucine residue, Leu; an L-leucine residue, Lys; an L-lysine residue, Met; an L-methionine residue, Phe; an L-phenylalanine residue, Pro; an L-proline residue, Ser; an L-serine residue, Thr; an L-threonine residue, Trp; an L-tryptophan residue, Tyr; an L-tyrosine residue, and Val; an L-valine residue. Moreover, in this specification, an amino acid sequence of a peptide is described according to a conventional method, in which an amino terminal (hereinafter, referred to as an n-terminal) of the sequence is positioned at the left side while a carboxyl terminal (hereinafter, referred to as a c-terminal) thereof is positioned at the right side.

More specifically, Patent Document 8 discloses the following contents.

Example 1 discloses the selection of the part to which the mutation for designing the amino acid sequence is introduced and the identification of the amino acid residue which is substituted in association with the mutant protein (hereinafter, referred to as "an improved protein G") which is obtained by introducing the mutation to the native-type amino acid sequence of the B1, B2 or B3 domain of the protein G represented by [SEQ ID NO. 1], [SEQ ID NO. 2] and [SEQ ID NO. 3], respectively.

Example 2 discloses that the amino acid sequences of the improved protein G represented by [SEQ ID NO. 4] to [SEQ ID NO. 19] were designed by utilizing information on the above-mentioned selected target parts for the mutation and the above-mentioned specified amino acid residues which would be substituted, that the amino acid sequences represented by [SEQ ID NO. 13] to [SEQ ID NO. 20] were finally selected as concrete amino acid sequences, and that improved proteins G with these sequences were then actually synthesized to evaluate the molecular properties.

Example 3 discloses the base sequence of the mutant protein using the base sequences of the nucleic acids ([SEQ ID NO. 13] to [SEQ ID NO. 20]) encoding the amino acid sequences of the improved proteins G and the base sequence of the Oxaloacetate decarboxylase alpha-subunit c-terminal domain (OXADac)([SEQ ID NO. 31].

Example 4 discloses that the plasmid vectors which contain the genes encoding the improved proteins G were synthesized by using the PG genes consisting of base sequences represented by [SEQ ID NO. 21] to [SEQ ID NO. 29], and that fusion proteins of the Oxaloacetate decarboxylase alpha-subunit c-terminal domains (OXADac) [SEQ ID NO. 31] and the mutant proteins were then produced with the Escherichia colis.

Example 5 discloses that the plasmid vectors which contain the genes encoding the improved proteins G were synthesized by using the various primers ([SEQ ID NO. 32] to [SEQ ID NO. 35]),, and that the Met addition improved proteins G were then produced with the Escherichia colis.

Example 6 discloses that purity of the improved proteins G was confirmed by the polyacrylamide gel electrophoresis method. Example 7 discloses that by measuring molecular weight of the improved proteins G with a MALDI-TOF type mass spectrometer, the produced proteins were identified. Example 8 discloses that by using the columns on which the OXADac-PG fusion proteins were immobilized, a pH gradient affinity chromatography was performed to determine pH for eluting a monoclonal antibody, so that antibody dissociation of the improved proteins G in the weakly acidic region was evaluated. Example 9 discloses that by using the columns on which the OXADac-PG fusion proteins were immobilized, a stepwise pH affinity chromatography was performed to examine the elution of the monoclonal antibodies at some pH, so that the antibody dissociation of the improved proteins G in the weakly acidic region was evaluated. Example 10 discloses that the binding dissociation of the mutant proteins (protein G mutants) was evaluated by the surface plasmon resonance (SPR) method. Example 11 discloses that the antibody-binding property of the mutant proteins, in the neutral region and a weakly acidic region in which more than 95 % of histidine residues were protonated, was evaluated by the surface plasmon resonance (SPR) method. Example 12 discloses that the thermal stability of the mutant protein was evaluated. Example 13 discloses that the single crystals of the mutant protein were produced and the stereoscopic structure was determined by an X-ray diffraction analysis.

Furthermore, Example 14 discloses that the production of the tandem-type multimer of the extracellular domain mutants of the protein G by using the two kinds of artificial synthesis plasmids incorporated with genes encoding the wild-type PG trimer (CGB01H-3D, SEQ ID NO. 36) or the tandem-type trimer of the mutant-type PG, which is the protein, (CGB19H-3D, SEQ ID NO. 37), in both of which the cysteine residue and the His tag had been added to the carboxyl terminal side; the preparation of the affinity chromatography column using the protein; and the purification of human IgG1 and IgG3 antibodies and the like using said column.

Example 15 discloses the comparison of the antibody-binding dissociation from the IgG1 type humanized monoclonal antibodies between the tandem-type multimer and the monomer of the extracellular domain mutants of the protein G.

Example 16 discloses the production of the monomer and tandem-type tetramer, and pentamer of the extracellular domain mutant of the protein G by using the three kinds of artificial synthesis plasmids for expression incorporated with genes encoding a monomer of the mutant-type PG (CGB19H-1D, FIG. 4, SEQ ID NO. 38), a tandem-type tetramer of the PG (CGB19H-4D, FIG. 4, SEQ ID NO. 39) and a tandem-type pentamer of the PG (CGB19H-5D, FIG. 4, SEQ ID NO. 40), in all of which the cysteine residue and the His tag had been added to the carboxyl terminal.

Example 17 discloses that by immobilizing the monomer and the tandem-type multimers of the extracellular domain mutant of the protein G to solid phases via the cysteine residues of the carboxyl terminal, the antibody-binding property of each mutant protein to the IgG1 type humanized monoclonal antibodies was compared and evaluated by the SPR method.

The present invention will be specifically explained with the following examples. The technical scope of the present invention is not restricted by these examples. Examples

### Example 1

### Production of the tandem-type dimers of the extracellular domain mutant of the protein G

In this example, the tandem-type dimers of the extracellular domain mutant of the protein G was produced, which comprise linkers with different length.

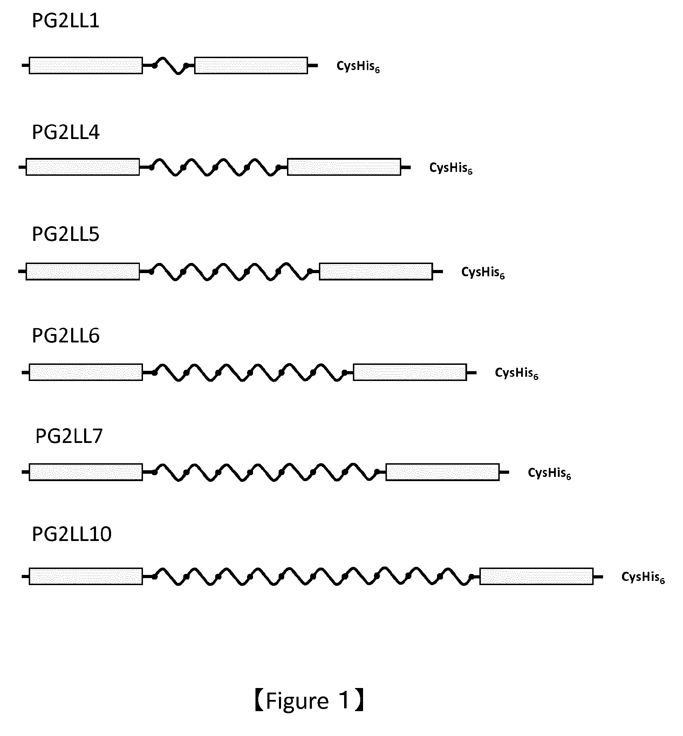

An expression plasmid was synthesized, which was incorporated with a gene encoding the dimer of the mutant-type PG (PG2LL10, FIG. 1, SEQ ID NO. 41) in which the cysteine residue and the His tag had been added to its carboxyl terminal side, and which comprises a linker with 60 amino acid residues comprising ten repeats of a unit of six amino acid residues of glycine and serine, and a restriction enzyme recognition site between domains. The expression plasmids encoding the dimers of the mutant-type PG (PG2LL7, PG2LL6, PG2LL5, PG2LL4, PG2LL1, FIG. 1, SEQ ID NOs. 42-46) having different lengths of the linkers were then prepared by means of a partial digestion reaction utilizing the restriction site present within the linker sequence, and the subsequent cyclization reaction in accordance with the method of Evers, et al. (Non-Patent Document 9). The escherichia colis strains for expression BL21(DE3) (Novagen) were transformed with the plasmids encoding the dimers of the mutant-type PG recombinant PG having each length of the linkers. The precultured transformants were subcultured into the 2YT mediums in 2 ml / 200 ml, and were shake-cultured to O.D.₆₀₀ = 0.8 to 1.0. After 0.5 mM of IPTG was added in order to express the target proteins, the transformants were further shake-cultured at 37 °C for two hours. The collected cell bodies were suspended in 10 ml of PBS and then were ultrasonically crushed before the filter sterilization, and the obtained solutions were treated as wholly protein solutions. After the recombinant PG were adsorbed on Ni Sepharose (GE Healthcare Bioscience) 2 ml columns and were washed with 20 mM of imidazole, purified proteins were eluted with 500 mM of imidazole.

### Example 2

### Evaluation of the antibody-binding property of the tandem-type dimers of the extracellular domain mutant of the protein G

In this example, by immobilizing the tandem-type dimers of the extracellular domain mutant (SEQ ID NO. 19) of the protein G having the different lengths of the linker to solid phases via the cysteine residues of the carboxyl terminal, the antibody-binding property of each mutant protein was compared and evaluated by the SPR method.

First, on measuring cells of the sensor chips CM-5 (GE Healthcare), each of the tandem-type dimers of the extracellular domain mutant of protein G (PG2LL1, PG2LL4, PG2LL5, PG2LL6, PG2LL7, PG2LL0) having different lengths of the linkers, which were produced in Example 1, were immobilized by a maleimide coupling method using EMCH (N-[ε-Maleimidocaproic acid] hydrazide, trifluoroacetic acid) (Thermo scientific), respectively. The immobilization amounts were adjusted so that proteins of the same mass were immobilized. Next, by dissolving the IgG1 type humanized monoclonal antibodies into running buffer solutions (10 mM HEPES pH 7.4, 150 mM NaCl, 0.005 % v/v Surfactant P20), 10µM and 1µM of sample solutions were prepared. The SPR measurement was performed at a reaction temperature of 25 °C with the Biacore T100 (GE Healthcare) (Fig. 2-1, Fig.2-2).

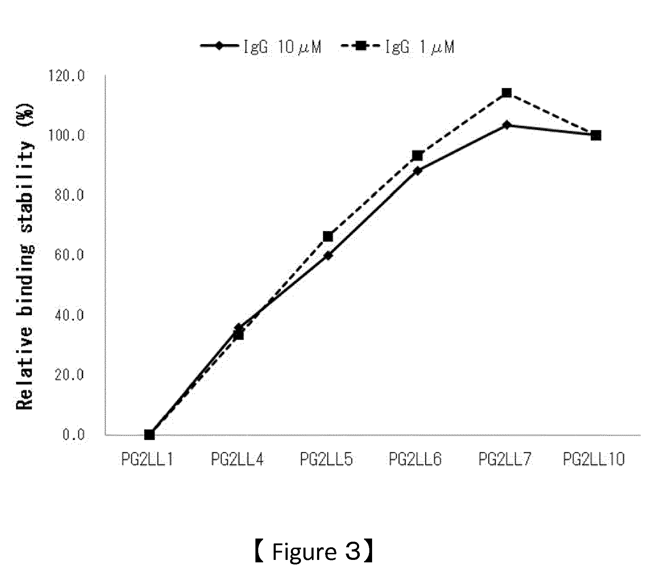

After 500 seconds when the sample antibodies were supplied to the chips on which the tandem-type dimers of the extracellular domain mutant of protein G having different lengths of the linkers of the present invention were immobilized at the same mass, amounts of the antibody bound on the chip were measured. It was observed that the antibody binding rate was increased as the length of the linker was increased, and that a maximum antibody binding rate was obtained in the chip on which the dimers (PG2LL7) having the linker of 42 residues was immobilized (Fig.3). It was also observed that the difference in the antibody binding rate among each length of the linkers was increased, as the concentration of the sample antibodies was decreased (Fig.3). From these results, it was found that there was an appropriate length of the linker for the tandem-type dimer according to the present invention to effectively bind to the sample antibodies.

### Example 3

### Production of the extracellular domain mutant of the protein G and Preparation of column using said protein

### (1) Expression of recombinant PG and purification

The escherichia colis strains were transformed with expression plasmids that were incorporated with a gene encoding the mutant-type PG (PG2LL7) constructed in Example 1, and a gene encoding the tandem-type tetramer PG, rPG-A4 described in Example 18 of Patent Document 8, in which the cysteine residue and the His tag had been added to its carboxyl terminal side (CGB19H-4D, SEQ ID NO. 39), respectively. The transformants were subcultured into the LB mediums , and were shake cultured to O.D.600 = 0.8 to 1.0. After 0.5 mM of IPTG was added in order to express the target proteins, the transformants were further shake-cultured at 37°C for three hours. The collected cell bodies were suspended in PBS and then were ultrasonically crushed before the centrifugation, and the resulting supernatant was treated as wholly protein solutions. After the recombinant PG were adsorbed on HisTrap FF (GE Healthcare Bioscience) 1 ml columns and were washed with 20 mM of imidazole, and eluted with 500 mM of imidazole to obtain purified proteins.

### (2) Immobilization of the recombinant PG and preparation of the column

Sepharose 4F FastFlow (GE Healthcare) was filtered through a glass filter washed with ultrapure water to give 10 ml of carrier. The resulting carrier was then transferred into a flask, mixed with 3 ml of 2 M sodium hydroxide aqueous solution and 4 g of butanediol diglycidyl ether to react at 25°C for four hours under shaking. Filtration through the glass filter and washing with ultrapure water gave an activated carrier. The activated carrier (1 ml) was taken on the glass filter and washed with a coupling buffer (0.1M sodium phosphate, 1.0 M sodium sulfate, 1 mM EDTA, pH 8.0). The activated carrier was then transferred into a flask, mixed with 2 ml of the coupling buffer and 1.5 ml of a solution comprising 5.4 mg/ml of the recombinant PG (PG2LL7), and shaken at 37°C and 150 rpm for 25 hours so as to immobilize the recombinant PG to the carrier via cysteine residue. The carrier was then filtered through the glass filter and washed with the coupling buffer. The carrier was then transferred into a flask, mixed with 3 ml of solution comprising 1M thioglycerol, 0.1M sodium phosphate, 1mM EDTA and pH 8.0, and shaken at 37°C and 150 rpm for 4 hours so as to mask the unreacted active groups. It was filtered through the glass filter, washed in three cycles alternatively with 15 ml of a washing solution 1 (0.1 M tris-HCl, 0.5M sodium chloride, pH8.0) and 15 ml of washing solution 2 (0.1 M acetic acid, 0.5M sodium chloride, pH4.0). The immobilized carrier (1 ml) was washed with ultrapure water and packed into Tricon 5/50 Column. Another column was prepared using rPG-A4 by the same operations as for PG2LL7.

### Example 4

### Antibody binding capacity of the recombinant PG-immobilized column

The recombinant PG-immobilized columns prepared in Example 3 were set to the liquid chromatography apparatus AKTAexplore (GE Healthcare Bioscience) and were equilibrated by supplying an absorbing buffer solution (20 mM phosphate buffer, 150 mM sodium chloride, pH 7.2) under a condition of 1 ml /min or 0.4 ml /min. The human IgG (Oriental Yeast Co., ltd) at 1 mg/ml was then injected until absorbance at 280 nm of the elution reached 15% of that of the injecting sample, followed by washing with the absorbing buffer solution, and the absorbing buffersolution was then replaced with 20 mM citric acid (pH2.4).

Dynamic binding capacity (DBC) was calculated on the amount of the sample that had been injected until the absorbance at 280 nm of the elution except non-absorbed components had reached 10% of that of the injecting sample. The DBC of each column is shown in Table 1. PG2LL7 and rPG-A4 showed almost the same DBC with each other, demonstrating that a high binding capacity could be obtained by increasing the length of the linker moiety so as to appropriately adjust the distance between the antibody-binding domains at each end instead of inserting further antibody-binding domains.

**[Table 1]**

| **Flow Rate** | **PG2LL7** | **rPG-A4** |
|---|---|---|
| **1.0 mL/min.** | **5.2 mg** | **5.3 mg** |
| **0.4 mL/min.** | **12.0 mg** | **12.3 mg** |

### Example 5

### pH gradient affinity chromatography

The recombinant PG-immobilized columns were set to the liquid chromatography apparatus AKTAexplore (GE Healthcare Bioscience) and were equilibrated by supplying an absorbing buffer solution (20 mM phosphate buffer, 150 mM sodium chloride, pH 7.2) under a condition of 1 ml /min. The human IgG (Oriental Yeast Co., ltd) at 1 mg/ml was then injected. After being washed with the absorbing buffer solution, the absorbing solution was then replaced with 20 mM citric acid (pH6.0) which was then continuously replaced with 20 mM citric acid (pH2.4) at a flow rate of 1.0ml/min over a period of 80 min. A peak of the elution of human IgG was about pH 3.9 and about pH 4.4 in the PG2LL7-immobilized column and rPG-A4-immobilized column, respectively (Fig.4), showing that although the PG2LL7-immobilized column had the DBC equal to that of the rPG-A4-immobilized column, the former column could enabled the elution under milder conditions.

### Industrial Applicability

Currently, the extracellular domain of the wild-type protein G is marketed as an affinity chromatography carrier for purifying an antibody and an inspection reagent for detecting an antibody, and is widely used in each field of life science. Moreover, with recent development of the antibody-related industries, including antibody medicine, demand for these products expands dramatically.

Accordingly, by replacing the wild-type proteins such as protein G and protein A and the conventional mutant protein (the improved protein) comprising their domain mutants with the protein of the present invention that can dissociate and elute the immunoglobulin in the weakly acidic region under the milder conditions and have the binding property at least comparable to that of the conventional proteins at the neutral region , the degradation of the antibody due to the elution with an acid can be decreased. Accordingly, the protein of the present invention significantly contributes to the technical development in the wide technical field where the antibodies derived from variety of animal species are treated.

## Claims

1. A protein constituted by linking plural domains having affinity for a protein comprising the Fc region of the immunoglobulin G (IgG) with linkers wherein an extended length to the maximum of the linker is 80 angstrom -240 angstrom.

2. The protein according to Claim 1 wherein the linker is a polypeptide linker.

3. The protein according to Claim 2 wherein the linker consists of a polypeptide of 22-66 amino acid residues.

4. The protein according to Claim 3 wherein the amino acid sequence of the linker consists of 4 to 10 times repeats of GlyGlySerGlyGlySer.

5. The protein according to Claim 4 wherein the amino acid sequence of the linker consists of 6 to 10 times repeats of GlyGlySerGlyGlySer.

6. The protein according to any one of Claims 1 to 5, wherein the domain is derived from protein G or protein A.

7. The protein according to Claim 6, wherein the domain is a mutant of any one of B1, B2 and B3 domains of a wild-type protein G.

8. The protein according to any one of Claims 1 to 7, wherein the domains are the same as one another.

9. The protein according to any one of Claims 1 to 8, which is a dimer consisting of the linked two domains.

10. The protein according to any one of the Claims 7 to 9, wherein the domain mutant is a mutant protein of B1 domain protein of the wild-type protein G,
the mutant protein consists of an amino acid sequence represented by (a) or of the amino acid sequence obtained by deleting, substituting, inserting or adding one or several amino acid residues in the amino acid sequence represented by (a),
the mutant protein has the binding property to the Fc region of immunoglobulin G, and the mutant protein has at least the binding property to the Fab region of immunoglobulin G and/or the binding property to the Fc region of immunoglobulin G in the weakly acidic region is decreased in comparison with a B 1 domain protein of the wild-type protein G. 
(a)AspThrTyrLysLeuIleLeuAsnGlyLysX11LeuLysGlyGluThrX17ThrGluAlaValX22AlaAla
X25AlaGluLysValPheLysX32TyrAlaX35X36X37GlyValX40GlyX42TrpThrTyrAspX47X48ThrLys
ThrPheThrValThrGlu
 (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or Leu; X47 is Asp or Pro; X48 is Ala, Lys or Glu; X22 is Asp; X25 is Thr; X32 is Gln; X40 is Asp; X42 is Glu; and X11 is Thr, and X17 is Thr simultaneously.)

11. The protein according to any one of the Claims 7 to 9, wherein the domain mutant is a mutant protein of B2 domain protein of the wild-type protein G,
the mutant protein consists of an amino acid sequence represented by (b) or of the amino acid sequence obtained by deleting, substituting, inserting or adding one or several amino acid residues in the amino acid sequence represented by (b),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B2 domain protein of the wild-type protein G. 
(b)ThrThrTyrLysLeuValIleAsnGlyLysX11LeuLysGlyGluThrX17ThrGluAlaValX22AlaAlaX
25AlaGluLysValPheLysX32TyrAlaX35X36X37GlyValX40GlyX42TrpThrTyrAspX47X48ThrLysT
hrPheThrValThrGlu
 (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; X42 is Glu; and X11 is Thr and X17 is Thr simultaneously.)

12. The protein according to any one of the Claims 7 to 9, wherein the domain mutant is a mutant protein of B3 domain protein of the wild-type protein G,
the mutant protein consists of an amino acid sequence represented by (c) or of the amino acid sequence obtained by deleting, substituting, inserting or adding one or several amino acid residues in the amino acid sequence represented by (c),
the mutant protein has the binding property to the Fc region of immunoglobulin G, and
the mutant protein has at least the binding property to the Fab region of immunoglobulin G and/or the binding property to the Fc region in the weakly acidic region is decreased in comparison with B3 domain protein of the wild-type protein G. 
(c)ThrThrTyrLysLeuValIleAsnGlyLysX11LeuLysGlyGluThrX17ThrLysAlaValX22AlaGluX
25AlaGluLysAlaPheLysX32TyrAlaX35X36X37GlyValX40GlyValTrpThrTyrAspX47X48ThrLysTh
rPheThrValThrGlu
 (In the amino acid sequence, X35 represents Asn or Lys; X36 represents Asp or Glu; X37 represents Asn, His or Leu; X47 represents Asp or Pro; X48 represents Ala, Lys or Glu; X22 represents Asp or His; X25 represents Thr or His; X32 represents Gln or His; X40 represents Asp or His; X11 represents Thr or Arg; and X17 represents Thr or Ile, respectively, with the proviso that a case is excluded where X35 is Asn or Lys; X36 is Asp or Glu; X37 is Asn or His; X47 is Asp or Pro; X48 is Ala, Lys or Glu; X22 is Asp; X25 is Thr; X32 is Gln; X40 is Asp; and X11 is Thr and X17 is Thr simultaneously.)

13. The protein according to any one of the Claims 7 to 9, wherein the domain mutant is a mutant protein of B 1 domain protein of the wild-type protein G,
the mutant protein consists of an amino acid sequence represented by (d) or of the amino acid sequence obtained by deleting, substituting, inserting or adding one or several amino acid residues in the amino acid sequence represented by (d),
the mutant protein has the binding property to the Fc region of immunoglobulin G, and
the mutant protein has the binding property to the Fab region of immunoglobulin G and/or the binding property to the Fc region in the weakly acidic region is decreased in comparison with B 1 domain protein of the wild-type protein G. 
(d)AspThrTyrLysLeuIleLeuAsnGlyLysX11LeuLysGlyGluThrX17ThrGluAlaValX22AlaAla
X25AlaGluLysValPheLysX32TyrAlaAsnAspAsnGlyValX40GlyX42TrpThrTyrAspAspAlaThrLysT
 hrPheThrValThrGlu (In the amino acid sequence, X22 represents Asp or His; X25 represents Thr or His; X32 represents Gln or His; X40 represents Asp or His; X42 represents Glu or His; XII represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

14. The protein according to any one of the Claims 7 to 9, wherein the domain mutant is a mutant protein of B2 domain protein of the wild-type protein G,
the mutant protein consists of an amino acid sequence represented by (e) or of the amino acid sequence obtained by deleting, substituting, inserting or adding one or several amino acid residues in the amino acid sequence represented by (e),
the mutant protein has the binding property to the Fc region of immunoglobulin G, and
the mutant protein has the binding property to the Fab region of immunoglobulin G and/or the binding property to the Fc region in the weakly acidic region is decreased in comparison with B2 domain protein of the wild-type protein G. 
(e)ThrThrTyrLysLeuValIleAsnGlyLysX11LeuLysGlyGluThrX17ThrGluAlaValX22AlaAlaX
25AlaGluLysValPheLysX32TyrAlaAsnAspAsnGlyValX40GlyX42TrpThrTyrAspAspAlaThrLysThr
PheThrValThrGlu
 (In the amino acid sequence, X22 represents Asp or His; X25 represents Thr or His; X32 represents Gln or His; X40 represents Asp or His; X42 represents Glu or His; X11 represents Thr or Arg; and X17 represents Thr or Ile, respectively, with the proviso that a case is excluded where X22 is Asp; X25 is Thr; X32 is Gln; X40 is Asp; X42 is Glu; and X11 is Thr and X17 is Thr simultaneously.)

15. The protein according to any one of the Claims 7 to 9, wherein the domain mutant is a mutant protein of B3 domain protein of the wild-type protein G,
the mutant protein consists of an amino acid sequence represented by (f) or of the amino acid sequence obtained by deleting, substituting, inserting or adding one or several amino acid residues in the amino acid sequence represented by (f),
the mutant protein has the binding property to the Fc region of immunoglobulin G, and
the mutant protein has the binding property to the Fab region of immunoglobulin G and/or the binding property to the Fc region in the weakly acidic region is decreased in comparison with B3 domain protein of the wild-type protein G. 
(f)ThrThrTyrLysLeuValIleAsnGlyLysX11LeuLysGlyGluThrX17ThrLysAlaValX22AlaGluX
25AlaGluLysAlaPheLysX32TyrAlaAsnAspAsnGlyValX40GlyValTrpThrTyrAspAspAlaThrLysThr
PheThrValThrGlu
 (In the amino acid sequence, X22 represents Asp or His; X25 represents Thr or His; X32 represents Gln or His; X40 represents Asp or His; X11 represents Thr or Arg; and X17 represents Thr or Ile, respectively, with the proviso that a case is excluded where X22 is Asp; X25 is Thr; X32 is Gln; X40 is Asp; and X11 is Thr and X17 is Thr simultaneously.)

16. The protein according to any one of the Claims 7 to 9, wherein the domain mutant is a mutant protein of B1 domain protein of the wild-type protein G,
the mutant protein consists of an amino acid sequence represented by (g) or of the amino acid sequence obtained by deleting, substituting, inserting or adding one or several amino acid residues in the amino acid sequence represented by (g),
the mutant protein has the binding property to the Fc region of immunoglobulin G, and
the mutant protein has the binding property to the Fc region in the weakly acidic region is decreased in comparison with B 1 domain protein of the wild-type protein G. 
(g)AspThrTyrLysLeuIleLeuAsnGlyLysThrLeuLysGlyGluThrThrThrGluAlaValX22AlaAlaX
25AlaGluLysValPheLysX32TyrAlaAsnAspAsnGlyValX40GlyX42TrpThrTyrAspAspAlaThrLysThr
PheThrValThrGlu
 (In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

17. The protein according to any one of the Claims 7 to 9, wherein the domain mutant is a mutant protein of B2 domain protein of the wild-type protein G,
the mutant protein consists of an amino acid sequence represented by (h) or of the amino acid sequence obtained by deleting, substituting, inserting or adding one or several amino acid residues in the amino acid sequence represented by (h),
the mutant protein has the binding property to the Fc region of immunoglobulin G, and
the mutant protein has the binding property to the Fc region in the weakly acidic region is decreased in comparison with B2 domain protein of the wild-type protein G. 
(h)ThrThrTyrLysLeuValIleAsnGlyLysThrLeuLysGlyGluThrThrThrGluAlaValX22AlaAlaX2
5AlaGluLysValPheLysX32TyrAlaAsnAspAsnGlyValX40GlyX42TrpThrTyrAspAspAlaThrLysThrP
heThrValThrGlu
 (In the amino acid sequence, X22 represents Asp or His; X25 represents Thr or His; X32 represents Gln or His; X40 represents Asp or His; and X42 represents Glu or His, respectively, with the proviso that a case is excluded where X22 is Asp; X25 is Thr; X32 is Gln; and X40 is Asp and X42 is Glu simultaneously.)

18. The protein according to any one of the Claims 7 to 9, wherein the domain mutant is a mutant protein of B3 domain protein of the wild-type protein G,
the mutant protein consists of an amino acid sequence represented by (i) or of the amino acid sequence obtained by deleting, substituting, inserting or adding one or several amino acid residues in the amino acid sequence represented by (i),
the mutant protein has the binding property to the Fc region of immunoglobulin G, and
the mutant protein has the binding property to the Fc region in the weakly acidic region is decreased in comparison with B3 domain protein of the wild-type protein G. 
(i)ThrThrTyrLysLeuValIleAsnGlyLysThrLeuLysGlyGluThrThrThrLysAlaValX22AlaGluX2
5AlaGluLysAlaPheLysX32TyrAlaAsnAspAsnGlyValX40GlyValTrpThrTyrAspAspAlaThrLysThrP
heThrValThrGlu
 (In the amino acid sequence, X22 represents Asp or His; X25 represents Thr or His; X32 represents Gln or His; and X40 represents Asp or His, respectively, with the proviso that a case is excluded where X22 is Asp; and X25 is Thr; X32 is Gln and X40 is Asp simultaneously.)

19. The protein according to any one of the Claims 7 to 9, wherein the domain mutant is a mutant protein consists of an amino acid sequence represented by (j):

(j)AspThrTyrLysLeuIleLeuAsnGlyLysThrLeuLysGlyGluThrThrThrGluAlaValAspAlaAlaTh
rAlaGluLysValPheLysHisTyrAlaAsnGluHisGlyValHisGlyHisTrpThrTyrAspProGluThrLysThrPheT
hrValThrGlu.

20. A protein that is a fusion protein consisting of a linked amino acid sequence of an amino acid sequence of the protein according to any one of the Claims 1 to 19 with an amino acid sequence of another protein.

21. The protein according to Claim 20, having an amino acid sequence represented by any one of SEQ ID NO. 41 to 45.

22. A nucleic acid encoding the protein according to any one of the Claims 1 to 21.

23. A recombinant vector comprising the nucleic acid according to Claim 22.

24. A transformant into which the recombinant vector according to Claim 23 is introduced.

25. A capturing agent for immunoglobulin G, or protein comprising the Fc or Fab region of the immunoglobulin G, wherein the protein according to any one of the Claims 1 to 21 is immobilized to a water-insoluble solid support.

26. An affinity chromatography comprising the capturing agent according to Claim 25 for the purification of immunoglobulin G, or the protein comprising the Fc or Fab region of the immunoglobulin G.

27. A method for the purification of immunoglobulin G, or the protein comprising the Fc or Fab region of the immunoglobulin G by means of the affinity chromatography for the purification according to Claim 26.
